(19) Europäisches Patentamt
European Patent Office
Office européen des brevets

(11) EP 1 144 353 B1

(12) **EUROPÄISCHE PATENTSCHRIFT**

(45) Veröffentlichungstag und Bekanntmachung des Hinweises auf die Patenterteilung:
**28.12.2005 Patentblatt 2005/52**

(21) Anmeldenummer: **99967928.5**

(22) Anmeldetag: **07.12.1999**

(51) Int Cl.$^7$: **C07C 45/86**

(86) Internationale Anmeldenummer:
**PCT/EP1999/009543**

(87) Internationale Veröffentlichungsnummer:
**WO 2000/034220 (15.06.2000 Gazette 2000/24)**

(54) **VERFAHREN ZUR STABILISIERUNG VON ETHYLENISCH UNGESÄTTIGTEN VERBINDUNGEN GEGEN UNERWUNSCHTE RADIKALISCHE POLYMERISATION**

METHOD FOR STABILIZING CHEMICAL COMPOUNDS CONTAINING AT LEAST ONE ETHYLENICALLY UNSATURATED BOND IN ORDER TO PREVENT UNDESIRABLE RADICAL POLYMERIZATION

PROCEDE POUR EMPECHER LA POLYMERISATION RADICALAIRE NON SOUHAITEE DE COMPOSES CHIMIQUES PRESENTANT AU MOINS UNE LIAISON ETHYLENIQUEMENT INSATUREE

(84) Benannte Vertragsstaaten:
**BE DE FR**

(30) Priorität: **08.12.1998 DE 19856565**

(43) Veröffentlichungstag der Anmeldung:
**17.10.2001 Patentblatt 2001/42**

(73) Patentinhaber: **BASF Aktiengesellschaft**
**67056 Ludwigshafen (DE)**

(72) Erfinder:
- **SUTORIS, Heinz, Friedrich**
  **D-67227 Frankenthal (DE)**
- **WAGENBLAST, Gerhard**
  **D-67157 Wachenheim (DE)**
- **SCHLIEPHAKE, Volker**
  **D-67105 Schifferstadt (DE)**
- **SCHRÖDER, Jürgen**
  **D-67071 Ludwigshafen (DE)**
- **MÜLLER-ENGEL, Klaus, Joachim**
  **D-76297 Stutensee (DE)**
- **KELLER, Harald**
  **D-67069 Ludwigshafen (DE)**
- **JAWOREK, Thomas**
  **D-67169 Kallstadt (DE)**

(56) Entgegenhaltungen:
**EP-A- 0 467 850** **EP-A- 0 765 856**
**WO-A-98/58891**

Bemerkungen:
Derzeit sind die WIPO-Publikationsdaten A3 nicht verfügbar.

EP 1 144 353 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Vorliegende Erfindung betrifft ein Verfahren zur Stabilisierung von als Reinsubstanz oder als Bestandteil von Gemischen vorliegenden chemischen Verbindungen, die wenigstens eine ethylenisch ungesättigte Gruppe aufweisen, gegen unerwünschte radikalische Polymerisation, bei dem man der Reinsubstanz oder dem Gemisch Polymerisationsinhibitoren zusetzt, die enthalten:

a) wenigstens ein Nitroxyl-Radikal (Inhibitoren a))
und

b) wenigstens eine das Element Phosphor chemisch gebunden enthaltende chemische Verbindung (Inhibitor b)).

Wenigstens eine ethylenisch ungesättigte Gruppe aufweisende chemische Verbindungen verfügen aufgrund ihrer ethylenisch ungesättigten Gruppen über eine ausgeprägte Neigung zur radikalischen Polymerisation. Dies ist insofern von Vorteil, als sich wenigstens eine ethylenisch ungesättigte Gruppe aufweisende chemishe Verbindungen dadurch in hervorragender weise zur gezielten Herstellung von Polymerisaten auf dem Weg der initiierten radikalischen Polymerisation eignen, wie sie z.B. zur Produktion von Klebstoffen benötigt werden. Gleichzeitig ist die ausgeprägte Neigung zur radikalischen Polymerisation aber insofern von Nachteil, als es sowohl bei der Lagerung als auch bei der chemischen und/oder physikalischen Bearbeitung (z.B. Destillation oder Rektifikation) der wenigstens eine ethylenisch ungesättigte Gruppe aufweisenden chemischen Verbindungen oder diese enthaltender Gemische, insbesondere unter der Einwirkung von Wärme und/oder Licht, zur unerwünschten, spontanen, radikalischen Polymerisation der wenigstens eine ethylenisch ungesättigte Gruppe aufweisenden chemischen Verbindungen kommen kann. Abgesehen davon, daß derartige unkontrollierte radikalische Polymerisationen der wenigstens eine ethylenisch ungesättigte Gruppe aufweisenden chemischen Verbindungen oder diese enthaltenden Gemische ein erhebliches Gefahrenpotential beinhalten (radikalische Polymerisationen verlaufen stark exotherm und als Konsequenz häufig explosionsartig), ziehen sie darüber hinaus vielfältige andere Folgewirkungen nach sich. Beispielsweise kann sich bei der Destillation von wenigstens eine ethylenisch ungesättigte Gruppe aufweisenden chemischen Verbindungen enthaltenden Gemischen in unerwünschter Weise gebildetes Polymerisat auf der Oberfläche des Verdampfers niederschlagen (dort ist die Neigung zur Polymerisatbildung infolge der hohen Temperaturen erhöht) und dadurch den Wärmeübergang in unerwünschter Weise mindern. Gebildetes Polymerisat kann aber auch die Einbauten in Rektifikationskolonnen verstopfen, was unerwünschte Druckverluste verursacht. Beides erfordert das Unterbrechen des Rektifikationsprozesses, um in aufwendiger Weise das gebildete Polymerisat zu entfernen.

Es ist daher allgemeine Praxis, sowohl wenigstens eine ethylenisch ungesättigte Gruppe aufweisenden chemischen Verbindungen als auch solche chemischen Verbindungen enthaltenden Gemischen sowohl bei der Lagerung als auch bei der chemischen und/oder physikalischen Bearbeitung Verbindungen zuzusetzen, die als Inhibitoren beziehungsweise Retarder einer radikalischen Polymerisation der wenigstens eine ethylenisch ungesättigte Gruppe aufweisenden chemischen Verbindungen wirken und dadurch diese chemischen Verbindungen gegen unerwünschte radikalische Polymerisation stabilisieren.

DE-A 197 26 671 offenbart Mischungen aus einer Aminverbindung und einem Hydroxylaminderivat zur Polymerisationshemmung.

Aus der US-A 5,322,960 ist bekannt, Estern der (Meth)acrylsäure als Polymerisationsinhibitoren ein Gemisch zuzusetzen, das u.a. ein Nitroxyl-Radikal (eine Verbindung, die wenigstens eine >N-O·-Gruppe aufweist) enthält. Als mögliches Nitroxyl-Radikal wird dabei u.a. auch 4,4',4"-Tris-(2,2,6,6-tetramethylpiperidinooxyl)phosphit genannt, das auch im Gemisch mit anderen Nitroxyl-Radikalen eingesetzt werden kann.

Die EP-A 685 447 empfiehlt ebenfalls die Mitverwendung von 4,4',4"-Tris-(2,2,6,6-tetramethylpiperidinooxyl) phosphit zur Inhibierung der radikalischen Polymerisation von (Meth)acrylsäure ((Meth)acryl- wird in dieser Schrift als verkürzte Schreibweise für "Acryl- und/oder Methacryl-" verwendet) und/oder deren Estern. In ähnlicher Weise empfiehlt die CN-A 1 052 847 die Mitverwendung von N-Oxyl-phosphit-estern als radikalische Polymerisationsinhibitoren. Nachteilig an diesen, Phosphor intramolekular chemisch gebunden enthaltenden, Polymerisationsinhibitoren ist, daß das Verhältnis von N-Oxyl-Gruppen und Phosphor nicht variabel ist.

Die DE-A 21 33 921 betrifft ein Verfahren zur Verhinderung der radikalischen Polymerisation von $\alpha,\beta$-monoethylenisch ungesättigten Carbonsäuren, bei dem man als Polymerisationsinhibitor eine Kombination von wenigstens einer hydroxylgruppenhaltigen Verbindung wie z.B. Kresol und wenigstens einer das Element Phosphor chemisch gebunden enthaltenden Verbindung wie z.B. Phosphorsäure oder Derivate derselben verwendet. Phosphorsäure enthält die Gesamtmenge des in ihr chemisch gebunden enthaltenen Phosphors mit der Oxidationszahl +5 chemisch gebunden. Gemäß der Lehre der DE-A 21 33 921 bewirkt die Kombination aus hydroxylgruppenhaltiger phenolischer Verbindung und das Element Phosphor chemisch gebunden enthaltender Verbindung in synergistischer Weise eine erhöhte Inhibitorwirkung.

Die DE-A 29 13 218 offenbart ein Verfahren zur Herstellung von (Meth)acrylsäureestern, bei dem zur Unterdrückung von radikalischer Polymerisation ein Gemisch aus wenigstens einem organischen Ester der phosphorigen Säure und wenigstens einem ein- oder zweiwertigen Phenol zugesetzt wird.

Die US-A 4,187,382 betrifft die Veresterung von organischen Diolen mit Acrylsäure. Sie empfiehlt, das Diol mit Triphenylphosphit vorzubehandeln, um so die Neigung des Reaktionsgemisches zur radikalischen Polymerisation zu verringern. Als Polymerisationsinhibitor wird die Verwendung eines konventionellen phenolischen Polymerisationsinhibitors empfohlen.

Die EP-A 810 196 empfiehlt als Polymerisationsinhibitoren für (Meth)acrylsäure und deren Ester die Kombination aus einem Nitroxyl-Radikal und einem tertiären Phosphin, in welchem der Phosphor mit drei Kohlenstoffatomen kovalent verbunden ist und die Oxidationszahl +3 aufweist. Gemäß der Lehre der EP-A 810 196 erhöht das Beisein des tertiären Phosphins in synergistischer Weise die Inhibitorwirkung von Nitroxyl-Radikalen. Die Mitverwendung von letzteren zur Stabilisierung von (Meth)acrylsäureestern gegen unerwünschte radikalische Polymerisation wird auch in der DE-A 19734171 empfohlen.

Die Aufgabe der vorliegenden Erfindung bestand darin, ein Verfahren zur Stabilisierung von als Reinsubstanz oder als Bestandteil von Gemischen vorliegenden chemischen Verbindungen, die wenigstens eine ethylenisch ungesättigte Gruppe aufweisen, gegen unerwünschte radikalische Polymerisation, zur Verfügung zu stellen, bei dem man der Reinsubstanz oder dem Gemisch Polymerisationsinhibitoren zusetzt, die wenigstens ein Nitroxyl-Radikal und wenigstens eine weitere chemische Verbindung enthalten, die die inhibierende Wirkung von Nitroxyl-Radikalen in synergistischer Weise zu erhöhen vermag.

Demgemäß wurde ein Verfahren zur Stabilisierung von als Reinsubstanz oder als Bestandteil von Gemischen vorliegenden chemischen Verbindungen, die wenigstens eine ethylenisch ungesättigte Gruppe aufweisen, gegen unerwünschte radikalische Polymerisation, bei dem man der Reinsubstanz oder dem Gemisch Polymerisationsinhibitoren zusetzt, die enthalten:

a) wenigstens ein Nitroxyl-Radikal (Inhibitoren a))
und

b) wenigstens eine das Element Phosphor chemisch gebunden enthaltene chemische Verbindung (Inhibitor b)),

gefunden, das dadurch gekennzeichnet ist, daß der wenigstens eine Inhibitor b) einerseits keine N-Oxyl-Gruppe und andererseits wenigstens einen Teil des Phosphors mit einer Oxidationszahl OZ $-3 \leq OZ \leq +5$ (häufig $+1 < OZ \leq +5$, oft $+3 \leq OZ \leq +5$) chemisch gebunden enthält, wobei dieser Phosphor gleichzeitig mit wenigstens einem Wasserstoff, Stickstoff, Sauerstoff und/oder Schwefel kovalent verbunden ist.

Bevorzugt sind dabei Inhibitoren b), deren Gesamtmenge an enthaltendem Phosphor der vorgenannten Definition genügt.

Unter der Oxidationszahl eines Atoms innerhalb einer kovalenten Verbindung bekannter Struktur soll hier eine Zahl mit positivem oder negativem Vorzeichen verstanden werden, die die Ladung angibt, welche das Atom haben würde, wenn man die bindenden Elektronenpaare derjenigen der kovalenten Bindungen, an denen es teilnimmt dem jeweils elektronegativeren Atom innerhalb der kovalenten Bindung zuteilt. Bei Elektronenpaaren aus kovalenten Bindungen zwischen zwei gleichen Atomen erhält jedes Atom ein Elektron.

Die Elektronegativität ist dabei in dieser Schrift ein auf Pauling zurückgehendes Maß für die Fähigkeit eines Atoms, in einer Kovalenzbindung Elektronen anzuziehen.

Die in dieser Schrift relevanten Elektronegativitäten sind diejenigen gemäß H.R. Christen, Grundlagen der allgemeinen und anorganischen Chemie, Verlag Sauerländer, Aarau, Diesterweg Salle, Frankfurt am Main (1973). Für die wichtigsten Elemente des Periodensystems weisen diese Elektronegativitäten die nachfolgenden Werte auf:

Be (1,5); B (2,0); H (2,1); C (2,5); Si (1,8); Ge (1,7); N (3,0); P (2,1); As (2,0); Sb (1,8); 0 (3,5); S (2,5); Se (2,4); Te (2,1); F (4,0); Cl (3,0); Br (2,8); J (2,4).

Erfindungsgemäß geeignete Inhibitoren b) sind z.B. die Phosphane der allgemeinen Formel $P_nH_{n+2}$ mit n = 1-7, der allgemeinen Formel $P_nH_n$ mit n= 3-10, der allgemeinen Formel $P_nH_{n-2}$ mit n= 4-10 sowie der allgemeinen Formel $P_nH_{n-4}$ mit n= 6-9.

Weitere erfindungsgemäß geeignete Inhibitoren b) sind die sich vom einfachsten Phosphan, dem $PH_3$, durch (z.B. partielle) Substitution der Wasserstoffatome durch Alkyl- und/oder Arylgruppen ableitenden primären und sekundären Phosphine sowie die quartären Phosphoniumbasen. Beispielhaft genannt seien das Methylphosphin ($CH_3PH_2$), das Dimethylphosphin ($(CH_3)_2PH$) und das Tetramethylphosphoniumhydroxid. Zweckmäßigerweise werden vorgenannte Alkylgruppen 1 bis 10 Kohlenstoffatome und vorgenannte Arylgruppen 6 bis 10 C-Atome aufweisen. D.h., Mono- und Di-phenylphosphin sind erfindungsgemäß ebenfalls geeignet. Ortho-phosphorsäuren der allgemeinen Formel

$H_3PO_n$ (n= 2, 3, 4, 5 und 6), die wasserärmeren Meta-phosphorsäuren der allgemeinen Formel $HPO_{n-1}$ (n= 3 und 4), die Disäuren $H_4P_2O_n$ (n= 4, 5, 6, 7 und 8) und die Polyphosphorsäuren mit drei und mehr Phosphoratomen je Molekül, z.B. diejenigen der allgemeinen Formel $H_{n+2}P_nO_{3n+1}$ (n= 3 bis 30), sowie die Salze (insbesondere die Alkalimetall- und Ammoniumsalze) und Ester mit organischen Alkoholen (insbesondere $C_1$- bis $C_8$-Alkanole) der vorgenannten Säuren sind gleichfalls erfindungsgemäß geeignete Inhibitoren b).

Beispielhaft seien die Ortho-phosphorsäure $H_3PO_4$, die Orthophosphorige Säure $H_3PO_3$, die Hypo-phosphorige Säure $H_3PO_2$ und die Hypo-diphosphorsäure $H_4P_2O_6$ genannt.

Das gleiche gilt für die bei vorsichtiger Oxidation von primären, sekundären und tertiären Alkylphosphinen entstehenden Alkylphosphinsäuren, Dialkylphosphinsäuren und Trialkylphosphinoxide sowie deren Arylanaloge und die entsprechenden Alkyl-Aryl-Mischformen. Auch die Salze (insbesondere die Alkalimetall- und Ammoniumsalze) und Ester mit organischen Alkoholen (insbesondere $C_1$- bis $C_8$-Alkanole) der Alkylphosphinsäuren und Dialkylphosphinsäuren sind als Inhibitoren b) geeignet.

Beispielhaft genannt seien die Methylphosphonsäure, die Dimethylphosphinsäure und das Trimethylphosphinoxid:

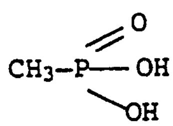 , 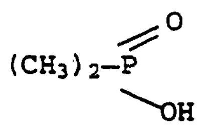 ,

$(CH_3)_3P=O$.

Außerdem eignen sich für das erfindungsgemäße Verfahren die Phosphonigsäure R*-P(OH)$_2$, mit R*= Alkyl (vorzugsweise $C_1$- bis $C_8$-Alkyl) oder Aryl (vorzugsweise Phenyl) und die Phosphinigsäure

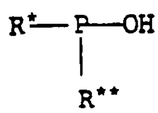 ,

mit R** unabhängig von R* dieselbe Bedeutung wie R*.

Das gleiche gilt für die Salze (insbesondere die Alkalimetall- und Ammoniumsalze) und die Ester mit organischen Alkoholen (insbesondere $C_1$- bis $C_8$-Alkanolen) dieser Säuren.

Verbindungen wie Pentaphenylphosphor sind erfindungsgemäß gleichfalls geeignete Inhibitoren b).

Auch Derivate der bisher beschriebenen sauerstoffhaltigen Phosphorverbindungen, bei denen ein oder mehrere O-Atome durch S oder -NR* ersetzt sind, sind erfindungsgemäß geeignete Inhibitoren b). Das gleiche gilt für nur aus P und S bestehende Verbindungen wie $P_4S_3$. Ferner kommen alle Phosphor enthaltenden, als Kunststoffstabilisatoren bekannten, Benzylphosphonate, Phosphite und Phosphonite als Inhibitoren b) in Betracht.

Zur Gruppe der Benzylphosphonate zählen beispielsweise Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat und Dioctadecyl-5-tert-butyl-4-hydroxy-3-methylbenzylphosphonat.

Zur Gruppe der Phosphite und Phosphonite zählen beispielsweise Triphenylphosphit, Diphenylalkylphosphit, Phenyldialkylphosphit, Tris(nonylphenyl)phosphit, Trilaurylphosphit, Trioctadecylphosphit, Distearylpentaerythritoldiphosphit, Tris(2,4-di-tert-butylphenyl)phosphit, Diisodecylpentaerythritoldiphosphit, Bis(2,4-di-tert-butylphenyl)pentaerythritoldiphosphit, Bis(2,6-di-tert-butyl-4-methylphenyl)-pentaerythritoldiphosphit, Diisodecyloxypentaerythritoldiphosphit, Bis(2,4-di-tert-butyl-6-methylphenyl)pentaerythritoldiphosphit, Bis(2,4,6-tris-(tert-butylphenyl))pentaerythritoldiphosphit, Tristearylsorbitoltriphosphit, Tetrakis(2,4-di-tert-butylphenyl)-4,4'-biphenylendiphosphonit, 6-Isooctyloxy-2,4,8,10-tetra-tert-butyl-12H-dibenz-[d,g]-1,3,2-dioxaphosphocin, 6-Fluoro-2,4,8,10-tetra-tert-butyl-12-methyl-dibenz[d,g]-1,3,2-dioxaphosphocin, Bis(2,4-di-tert-butyl-6-methylphenyl)methylphosphit und Bis(2,4-di-tert-butyl-6-methylphenyl)ethylphosphit.

Vorzugsweise werden als Inhibitoren b) erfindungsgemäß u.a. orthophosphorige Säure und deren Ester verwendet. Mit Vorteil werden dabei Phosphite der allgemeinen Formel (0)

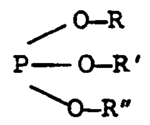 (0),

verwendet,
wobei R, R', R" gleich oder verschieden sein können und organische Reste, insbesondere $C_1$-$C_8$-Alkyl, Hydroxyalkyl mit 2 bis 4 C-Atomen, Halogenalkyl, insbesondere Chloralkyl mit 2 bis 4 C-Atomen, Aryl, insbesondere Phenyl oder durch $C_1$-$C_4$-Alkyl substituiertes Aryl (insbesondere durch $C_1$-$C_4$-Alkyl substituiertes Phenyl) bedeuten. Auch können zwei der drei organischen Reste R, R' und R" gemeinsam mit dem Phosphor und den beiden Sauerstoffatomen einen Heterocyclus (z.B. 5- oder 6-atomig) bilden.

Namentlich genannt seien Trimethyl-, Triethyl-, Tributyl-, Trihexyl-, Trioctyl-, Triphenyl, Tri-p-kresyl-, Trixylyl-, Tritolyl- und Tri-β-chlorethylphosphit. Aber auch Dimethyl-, Diethyl-, Dibutyl-, Dioctyl-, Diphenyl-, Ditolyl- und Dixylylphosphite sind erfindungsgemäß geeignete Inhibitoren b). Unter den Warenzeichen Irgafos® oder Ultranox® vermarktete Inhibitoren b) sind z.B.

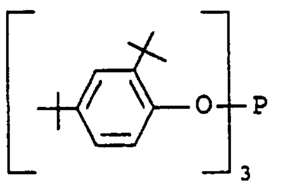 (Irgafos 168),

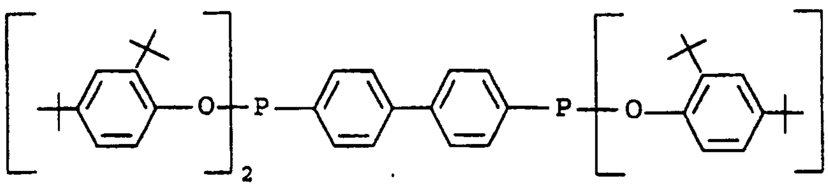 (Irgafos P-EPQ)

und

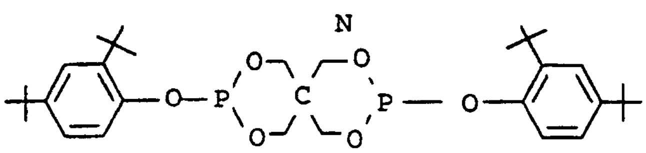 (Ultranox 626) .

Selbstredend kommen als erfindungsgemäß geeignete Inhibitoren b) auch Phosphide in Betracht. Natürlich können auch Gemische aus den genannten Inhibitoren b) für das erfindungsgemäße Verfahren verwendet werden.

Als erfindungsgemäß geeignete Nitroxyl-Radikale (auch als N-Oxyl-Radikale bezeichnet) kommen erfindungsgemäß prinzipiell alle Verbindungen in Betracht, die wenigstens eine >N-O·-Gruppe aufweisen. Das sind unter anderem alle in der DE-A 19734171 beschriebenen Nitroxyl-Radikale. Die Nitroxyl-Radikale können auch in situ aus anderen Verbindungen erzeugt werden, wie z.B. durch H-Abstraktion aus Hydroxylaminen oder durch Addition von C-Radikalen an Nitrone (vgl. H. Zweifel in "Stabilization of Polymeric Materials, Springer Verlag, Berlin/Heidelberg (1998), S. 52"). Sie können aber auch aus aromatischen Aminen die sich vom Anilin oder Phenylendiamin ableiten in situ erzeugt werden (vgl. vorgenannte Literatur S. 48). Selbstverständlich kommen aber auch alle diejenigen Nitroxyl-Radikale in Betracht, die in derjenigen Literatur beschrieben sind, die in der DE-A 19734171 zitiert wird. D.h., als erfindungsgemäß geeignete Nitroxyl-Radikale kommen vor allem diejenigen in Betracht, die sich von einem sekundären Amin ableiten, welches keine Wasserstoffatome an den α-C-Atomen trägt (d.h., die N-Oxyl-Gruppen leiten sich von entsprechenden sekundären Aminogruppen ab). Unter diesen eignen sich vor allem jene N-Oxyl-Radikale, die in der EP-A 135280, der älteren Anmeldung DE-A 19651307, der US-A 5,322,912, der US-A 5,412,047, der US-A

4,581,429, der DE-A 1618141, der CN-A 1052847, der US-A 4,670,131, der US-A 5,322,960, der älteren Anmeldung DE-A 19602539, der EP-A 765856, der WO 98/30601 und der JP-A 5/320217 genannt sind.

Solche geeigneten, sich von einem sekundären Amin ableitenden, stabilen N-Oxyl-Radikale sind z.B. jene der allgemeinen Formel I

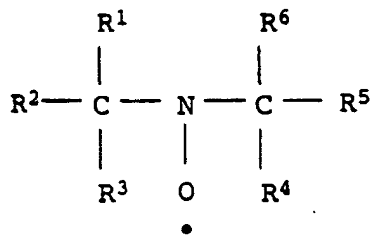 (I),

mit

| | |
|---|---|
| $R^1$,$R^2$,$R^5$ und $R^6$ = | dieselben oder verschiedene gerad- oder verzweigtkettige, gegebenenfalls substituierte Alkylgruppen und |
| $R^3$ und $R^4$ = | dieselben oder verschiedene gerad- oder verzweigtkettige, gegebenenfalls substituierte Alkylgruppen oder |
| $R^3CNCR^4$ = | eine, gegebenenfalls substituierte, zyklische Struktur. |

Als erfindungsgemäß geeignete Verbindungen I kommen insbesondere jene in Betracht, die in der EP-A 135 280, der älteren Anmeldung DE-A 19651307, der US-A 5,322,912, der US-A 5,412,047, der US-A 4,581,429, der DE-A 16 18 141, CN-A 1052847, US-A 4,670,131, US-A 5,322,960 sowie der älteren Anmeldung DE-A 19602539 genannt sind.

Beispiele dafür sind jene stabilen N-Oxyl-Radikale der allgemeinen Formel I, bei welchen $R^1$, $R^2$, $R^5$ und $R^6$ für (gleiche oder verschiedene) $C_1$- bis $C_4$-Alkylgruppen wie Methyl-, Ethyl-, n-Propyl-,iso-Propyl-, n-Butyl-, iso-Butyl- oder tert.-Butyl-, lineares oder verzweigtes Pentyl-, Phenyl- oder substituierte Gruppen hiervon und $R^3$ und $R^4$ für (gleiche oder verschiedene) $C_1$- bis $C_4$-Alkylgruppen wie Methyl-, Ethyl-, n-Propyl-, iso-Propyl-, n-Butyl-, iso-Butyl- oder tert.-Butyl-, lineares oder verzweigtes Pentyl-, substituierte Gruppen hiervon oder gemeinsam mit CNC die zyklische Struktur

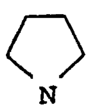 oder 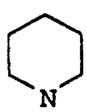 oder 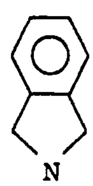 oder 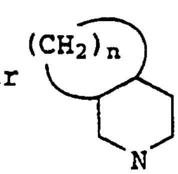 oder 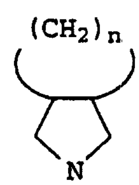

mit n gleich einer ganzen Zahl von 1 bis 10 (häufig 1 bis 6), einschließlich substituierter derartiger zyklischer Strukturen, stehen. Als beispielhafte Vertreter seien 2,2,6,6-Tetramethyl-1-oxyl-piperidin, 2,2,5,5-Tetramethyl-1-oxyl-pyrrolidin und 4-Oxo-2,2,6,6-tetramethyl-1-oxyl-piperidin genannt.

Die N-Oxyl-Radikale I lassen sich aus den entsprechenden sekundären Aminen durch Oxidation, z.B. mit Wasserstoffperoxid, herstellen. In der Regel sind sie als Reinsubstanz darstellbar.

Zu den erfindungsgemäß geeigneten N-Oxyl-Radikalen I zählen insbesondere Piperidin- oder Pyrrolidin-N-Oxyle und Di-N-Oxyle der nachstehenden allgemeinen Formeln II bis IX:

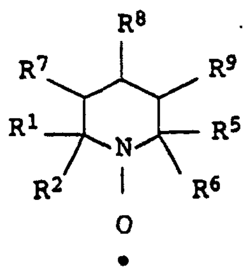
(II),

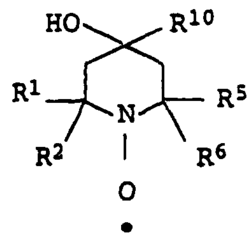
(III),

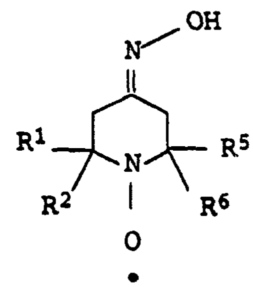
(IV),

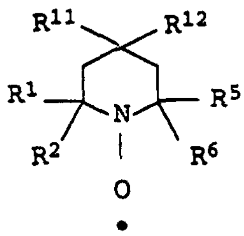
(V),

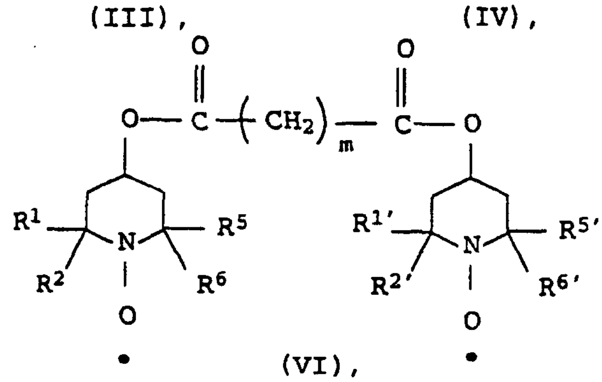
(VI),

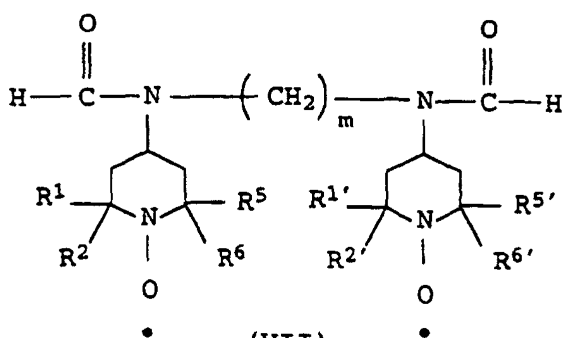
(VII),

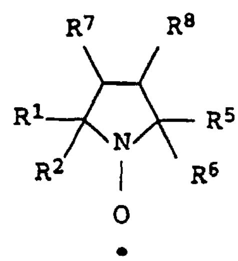
(VIII),

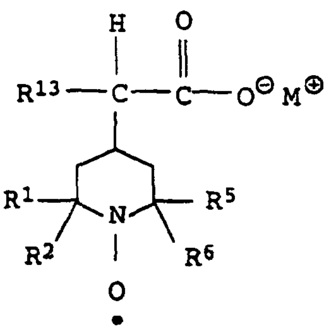
(IX),

mit

m = 2 bis 10,

$R^7$,$R^8$,$R^9$ = unabhängig voneinander —H,

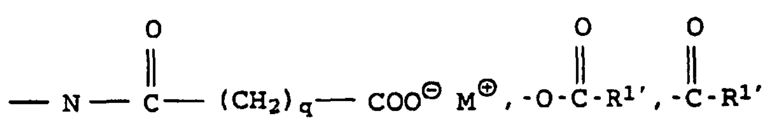

$—NH_2$,

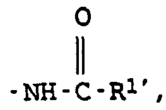, 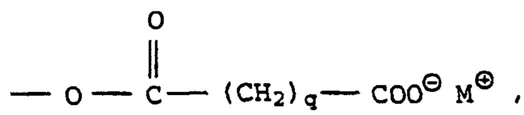

$—COO^{\ominus} M^{\oplus}$, $—SO_3^{\ominus} M^{\oplus}$, $—PO_3^{\ominus} M^{\oplus}$, $—O—PO_3^{2\ominus} M_2^{\oplus}$, $—O—SO_3^{\ominus} M^{\oplus}$, —OH,

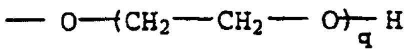

oder

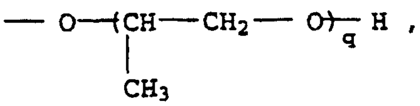

$M^{\oplus}$= ein Wasserstoff- oder ein Alkalimetallion,

q = eine ganze Zahl von 1 bis 10,

$R^{1'}$,$R^{2'}$,$R^{5'}$,$R^{6'}$ = unabhängig voneinander und unabhängig von $R^1$, $R^2$, $R^5$, $R^6$ dieselben Gruppen wie $R^1$,

$R^{10}$ = $C_1$- bis $C_4$-Alkyl, $-CH=CH_2$, -C≡CH, -CN,

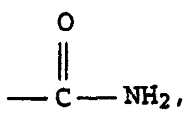

$-COO^{\ominus} M^{\oplus}$, $-COOCH_3$ oder $-COOC_2H_5$,

$R^{11}$ = ein organischer Rest, der wenigstens eine primäre, sekundäre (z.B. $-NHR^1$) oder tertiäre Aminogruppe (z.B. $-NR^1R^2$) oder wenigstens eine Ammoniumgruppe $-N^{\oplus}R^{14}R^{15}R^{16}X^{\ominus}$ aufweist, mit $X^{\ominus}$ = $F^{\ominus}$, $Cl^{\ominus}$, $Br^{\ominus}$, $HSO_4^{\ominus}$, $SO_4^{2\ominus}$, $H_2PO_4^{\ominus}$, $HPO_4^{2\ominus}$ oder $PO_4^{3\ominus}$ und $R^{14}$, $R^{15}$, $R^{16}$ voneinander unabhängige organische Reste (z.B. unabhängig voneinander und unabhängig von $R^1$ dieselben Gruppen wie $R^1$),

$R^{12}$ = unabhängig von $R^{11}$ dieselben Gruppen wie $R^{11}$ oder -H, -OH, $C_1$- bis $C_4$-Alkyl, $-COO^{\ominus}M^{\oplus}$, -C≡CH,

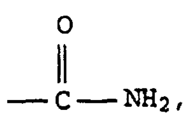, 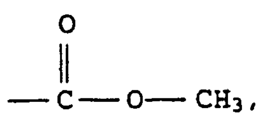, 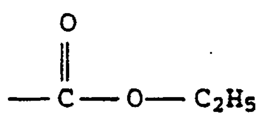

oder hydroxysubstituiertes $C_1$- bis $C_4$-Alkyl (z.B. hydroxyethyl oder hydroxypropyl) oder

$R^{11}$, $R^{12}$ = gemeinsam den Sauerstoff einer Carbonylgruppe und

$R^{13}$ = —H, $—CH_3$ oder

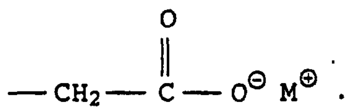

Vorzugsweise ist $R^1 = R^2 = R^5 = R^6 = R^{1'} = R^{2'} = R^{5'} = R^{6'} = -CH_3$.

Als beispielhafte Vertreter erfindungsgemäß geeigneter N-Oxyl-Radikale seien 1-Oxyl-2,2,6,6-tetramethylpiperidin, 1-Oxyl-2,2,6,6-tetramethylpiperidin-4-ol, 1-Oxyl-2,2,6,6-tetramethyl-4-methoxypiperidin, 1-Oxyl-2,2,6,6-tetramethyl-4-ethoxypiperidin, 1-Oxyl-2,2,6,6-tetramethyl-4-trimethylsiloxypiperidin, 1-Oxyl-2,2,6,6-tetramethylpiperidin-4-on, 1-Oxyl-2,2,6,6-tetramethylpiperidin-4-yl-acetat, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis(1-oxyl-2,2,6,6-tetramethylpiperidin-4-yl)-succinat, Bis (1-oxyl-2,2,6,6-tetramethylpiperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-n-butylmalonat, Bis(1-oxyl-2,2,6,6-tetramethylpiperidin-4-yl)-phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethylpiperidin-4-yl)-hexahydroterephthalat, N,N'-Bis(1-oxyl-2,2,6,6-tetramethylpiperidin-4-yl)-adipinamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-[N-butyl-N-(1-oxyl-2,2,6,6-tetramethylpiperidin-4-yl)]-s-triazin, N,N'-Bis(1-oxyl-2,2,6,6-tetramethylpiperidin-4-yl)-N,N'-bis-formyl-1,6-diaminohexan, 4,4'- Ethylen-bis(1-oxyl-2,2,6,6-tetramethylpiperazin-3-on) und Tris-(2,2,6,6-tetramethyl-1-oxyl-piperidin-4-yl)phosphit sowie zusätzlich noch 1-Oxyl-2,2,6,6-tetramethyl-1,2,3,6-tetrahydropyridin genannt.

Die Verbindungen (VI) und (VII) können gemäß US-A 4665185 (z.B. Bsp. 7) sowie DE-A 19510184 erhalten werden.

Weitere geeignete beispielhafte Vertreter sind:

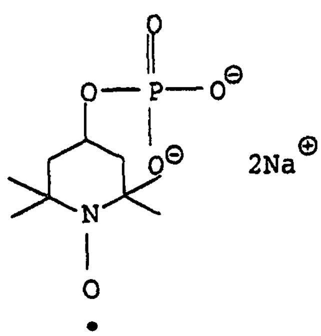

Sunamoto, Junzo; Akiyoshi, Kuzunari, Kihara, Tetsuji; Endo, Masayuki, BCS JA 8, Bull. Chem. Soc. Jpn., EN, 65, 4, 1992, S. 1041 - 1046;

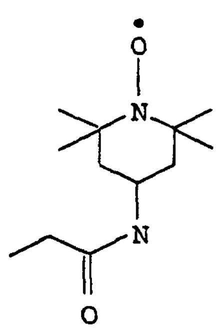

Beilstein Registry Number 6926369 ($C_{11}H_{22}N_3O_2$);

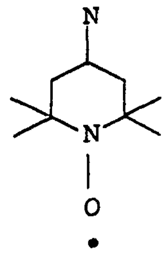

Beilstein Registry Number 6498805 (4-Amino-2,2,6,6-tetramethyl-1-oxylpiperidin);

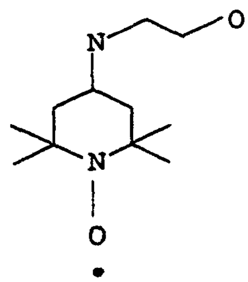

Beilstein Registry Number 6800244 ($C_{11}H_{23}N_2O_2$);

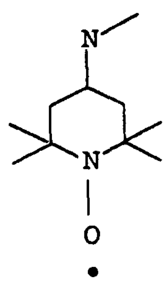

Beilstein Registry Number 5730772 (N-Methyl-4-amino-2,2,6,6-tetramethyl-1-oxyl-piperidin;

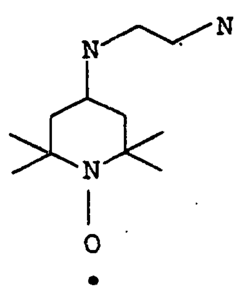

Beilstein Registry Number 5507538 (2,2,6,6-Tetramethyl-4-(2-amino-ethylamino)-1-oxyl-piperidin);

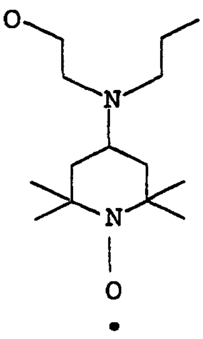

Beilstein Registry Number 4417950 (4<Bis(2-hydroxyethyl)>-amino-2,2,6,6-tetramethyl-1-oxyl-piperidin);

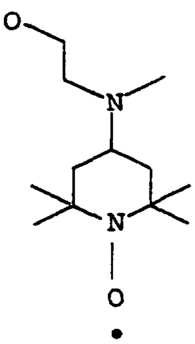

Beilstein Registry Number 4396625 ($C_{12}H_{25}N_2O_2$);

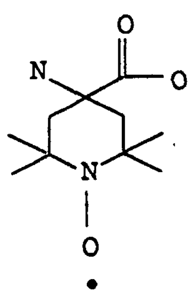

Beilstein Registry Number 4139900 (4-Amino-2,2,6,6-tetramethyl-4-carboxy-1-oxyl-piperidin);

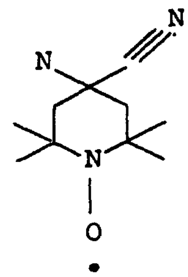

Beilstein Registry Number 4137088 (4-Amino-4-cyano-2,2,6,6-tetramethyl-1-oxyl-piperidin);

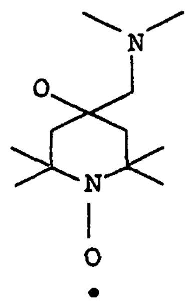

Beilstein Registry Number 3942714 ($C_{12}H_{25}N_2O_2$);

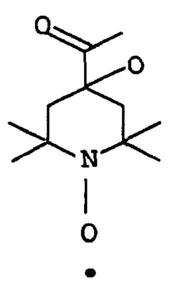

Beilstein Registry Number 1468515 (2,2,6,6-Tetramethyl-4-hydroxy-4-acetyl-1-oxyl-piperidin):

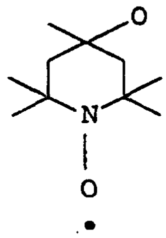

Beilstein Registry Number 1423410 (2,2,4,6,6-Pentamethyl-4-hydroxy-1-oxyl-piperidin);

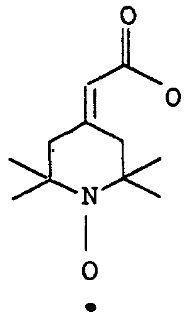

Beilstein Registry Number 6205316 (4-Carboxymethylen-2,2,6,6-tetramethyl-1-oxyl-piperidin);

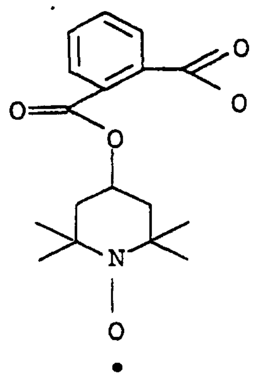

Beilstein Registry Number 1395538 (4-<2-Carboxy-benzoyloxy>-2,2,6,6-tetramethyl-1-oxyl-piperidin);

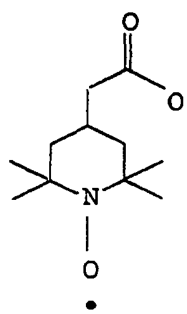

Beilstein Registry Number 3546230 (4-Carboxymethyl-2,2,6,6-tetramethyl-1-oxyl-piperidin);

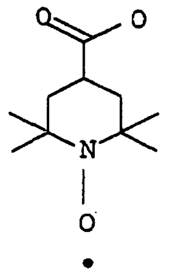

Beilstein Registry Number 3949026 (4-Carboxyl-2,2,6,6-tetramethyl-1-oxyl-piperidin);

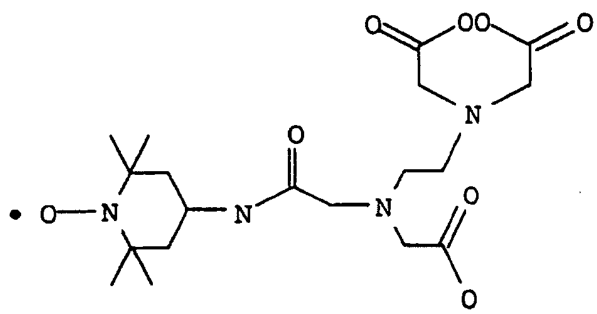

Beilstein Registry Number 4611003 (Ethylendiamintetraessigsäuremono(1-oxy-2,2,6,6-tetramethylpiperidinyl-4-amid);

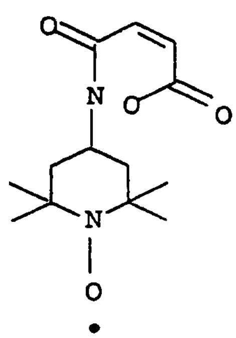

Beilstein Registry Number 5961636 ($C_{13}H_{21}N_2O_4$)

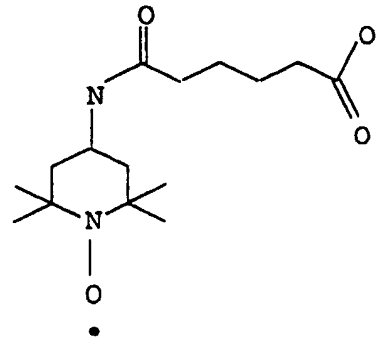

Beilstein Registry Number 5592232 ($C_{15}H_{27}N_2O_4$);

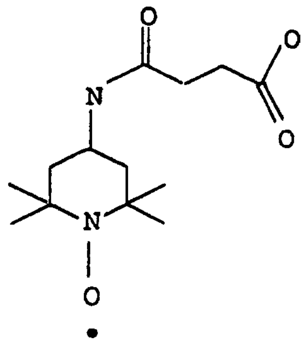

Beilstein Registry Number 5080576 (Bernsteinsäure-N-(2,2,6,6-tetramethyl-1-oxyl-4-piperidinyl)-monoamid);

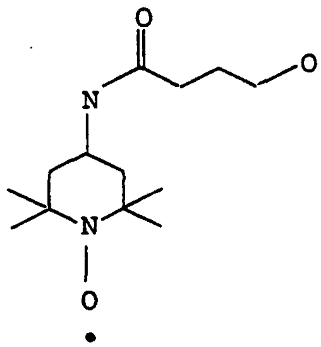

Beilstein Registry Number 5051814 (4-(4-Hydroxybutanoylamino)-2,2,6,6-tetramethyl-1-oxyl-piperidin);

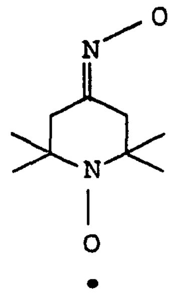

Beilstein Registry Number 4677496 (2,2,6,6-Tetramethyl-4-oximino-1-oxyl-piperidin);

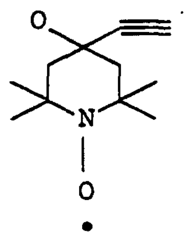

Beilstein Registry Number 1451068 ($C_{11}H_{18}NO_2$);

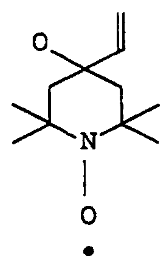

Beilstein Registry Number 1451075 ($C_{11}H_{20}NO_2$);

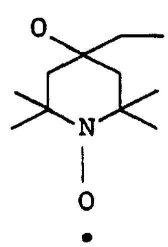

Beilstein Registry Number 1423698 (4-Ethyl-4-hydroxy-2,2,6,6-tetramethyl-1-oxyl-piperidin);

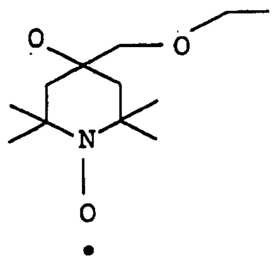

Beilstein Registry Number 5509793 (4-Ethoxymethyl-4-hydroxy-2,2,6,6 -tetramethyl-1-oxyl-piperidin);

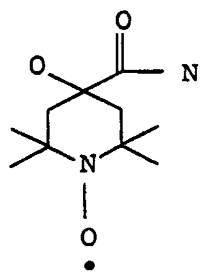

Beilstein Reqistry Number 3960373 ($C_{10}H_{19}N_2O_3$);

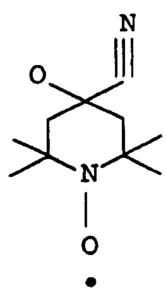

Beilstein Registry Number ($C_{10}H_{17}N_2O_2$);

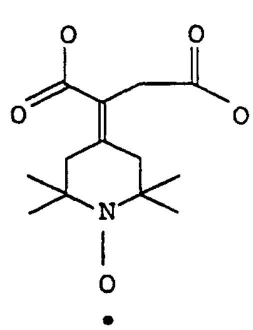

Beilstein Registry Number 3985130 (2,2,6,6-Tetramethyl-1-oxyl-4-piperidyliden)-bernsteinsäure):

Weitere erfindungsgemäß geeignete N-Oxyl-Radikale sind diejenigen, die in der WO 98/44008, der WO

97/46593 und der DE-A 19743396 offenbart werden.

Selbstverständlich können erfindungsgemäß auch Gemische von N-Oxyl-Radikalen angewendet werden.

In der Regel wird man bei dem erfindungsgemäßen Verfahren der als Reinsubstanz oder als Bestandteil von Gemischen vorliegenden, wenigstens eine ethylenisch ungesättigte Gruppe aufweisenden, chemischen Verbindung 1 bis 1000 gew.-ppm, häufig 50 bis 500 gew.-ppm (bezogen auf die enthaltene Menge der wenigstens einen wenigstens eine ethylenisch ungesättigte Gruppe aufweisenden chemischen Verbindung) an wenigstens einem Inhibitor a) zusetzen.

Das molare Verhältnis von erfindungsgemäß zuzusetzenden Inhibitoren a) zu erfindungsgemäß zuzusetzenden Inhibitoren b) beträgt normalerweise 1:100 bis 100:1, häufig 10:90 bis 10:10.

Selbstverständlich kann das im Rahmen des erfindungsgemäßen Verfahrens zuzusetzende Inhibitorsystem neben Inhibitoren a) und Inhibitoren b) noch weitere eine radikalische Polymerisation inhibierende Bestandteile umfassen.

Beispiele für solche sonstigen radikalischen Polymerisationsinhibitoren sind organische Nitrosoverbindungen wie N-Nitrosoarylamine oder die Nitrosogruppe unmittelbar an ein Kohlenstoffatom eines aromatischen Kerns gebunden aufweisende Nitrosoverbindungen.

Beispielhaft genannt seien Nitrosophenole wie 4-Nitrosophenol, aber auch Nitrosobenzol. Weitere zu nennende sonstige Polymerisationsinhibitoren sind die in der DE-A 19734171 genannten p-Phenylendiamine, phenolische Verbindungen wie Hydrochinon oder dessen Methylether oder Verbindungen wie Phenothiazin.

Günstig ist, daß das erfindungsgemäß zu verwendende Inhibitorgemisch seine Wirksamkeit auch im Beisein von molekularem Sauerstoff entfaltet.

Als wenigstens eine monoethylenisch ungesättigte Gruppe aufweisende chemische Verbindungen, bei denen das erfindungsgemäße Verfahren angewendet werden kann, kommen u.a. Verbindungen in Betracht wie die Olefine, z.B. Isobuten, Ethylen, Propylen, vinylaromatische Monomere wie Styrol, $\alpha$-Methylstyrol, o-Chlorstyrol oder Vinyltoluole, $C_4$-$C_8$-konjugierte Diene wie Butadien oder Isopren, Ester aus Vinylalkohol und 1 bis 18 C-Atome aufweisenden Monocarbonsäuren wie Vinylacetat, Vinylpropionat, Vinyl-n-butyrat, vinyllaurat und Vinylstearat.

Insbesondere ist das erfindungsgemäße Verfahren aber im Fall von 3 bis 6 C-Atome aufweisenden $\alpha,\beta$-monoethylenisch ungesättigten Mono- und Dicarbonsäuren, wie insbesondere Acrylsäure, Methacrylsäure, Maleinsäure, Fumarsäure und Itaconsäure, die Ester aus den vorgenannten Carbonsäuren und 1 bis 12, vielfach 1 bis 8 und häufig 1 bis 4 C-Atome aufweisenden Alkanolen, wie insbesondere Acrylsäure- und Methacrylsäuremethyl-, -ethyl-, -n-butyl-, -iso-butyl-, -tert.-butyl- und -2-ethylhexylester, Maleinsäuredimethylester oder Maleinsäure-di-n-butylester, geeignet. Das erfindungsgemäße Verfahren eignet sich aber auch im Fall der Vorläuferaldehyde, Nitrile und Amide der vorgenannten 3 bis 6 C-Atome aufweisenden $\alpha,\beta$-monoethylenisch ungesättigten Mono- und Dicarbonsäuren wie z.B. Acrolein, Methacrolein, Acrylnitril, Methacrylnitril, Acrylamid und Methacrylamid. Es ist aber auch anwendbar im Fall von Monomeren wie vinylsulfonsäure und N-Vinylpyrrolidon.

Das erfindungsgemäße Verfahren ist sowohl zur Lagerstabilisierung als auch für die Prozeßstabilisierung (Herstellung, Reinigung (z.B. Destillation) und chemische Umsetzung) von wenigstens eine monoethylenisch ungesättigte Gruppe aufweisenden Verbindungen geeignet. Letzteres gilt auch für destillative Behandlungen bei Temperaturen von 50 bis 300°C, häufig 50 bis 200°C oder auch 50 bis 150°C.

Insbesondere eignet sich das erfindungsgemäße Verfahren zur Stabilisierung bei der destillativen (rektifikativen) Behandlung von (Meth)acrylsäureestern (insbesondere der vorgenannten beispielhaften Vertreter), bei deren destillativer oder rektifikativer Abtrennung aus Produktgemischen, wie sie als Ergebnis einer säurekatalysierten (homogen und/oder heterogen) Veresterung von (Meth)acrylsäure mit Alkoholen, insbesondere Alkanolen (insbesondere $C_1$- bis $C_{12}$- bzw. $C_1$- bis $C_8$-Alkanolen) vor und/oder nach Abtrennung des Säurekatalysator vorliegen.

Es eignet sich aber auch zur Stabilisierung von vorgenannte (Meth)acrylsäureester enthaltenden Gemischen, die weder den sauren Veresterungskatalysator noch Acryl- oder Methacrylsäure selbst enthalten. Solche (Meth)acrylsäureester enthaltenden Gemische bilden beispielsweise vorgenannte Veresterungsproduktgemische nach z.B. extraktiver (z.B. mittels Wasser und/oder wäßriger Alkalilauge) und/oder rektifikativer Abtrennung des Säurekatalysators sowie nach entsprechender Abtrennung der überschüssigen (Meth)acrylsäure.

Die Stabilisierung eines einer Destillation (Rektifikation) unterworfenen (Meth)acrylsäureester enthaltenden Gemisches kann in einfacher Weise so erfolgen, daß man die erfindungsgemäß zuzusetzenden Inhibitoren dem Gemisch bereits vor der Destillation (Rektifikation) zugibt. Die Zugabe kann auch in den Zulauf zur Destillations(Rektifikations)kolonne erfolgen. In beiden vorgenannten Fällen kann man zweckmäßigerweise zusätzlich zur Stabilisierung der Kolonne Inhibitorzugabe auf den Kopf der Kolonne vornehmen. Selbstverständlich kann auch die Gesamtstabilisierung ausschließlich über eine Inhibitorzugabe auf den Kolonnenkopf vorgenommen werden.

Selbstredend können beim erfindungsgemäßen Verfahren die Inhibitoren a) und Inhibitoren b) zeitlich nacheinander, simultan oder auch bereits vorgemischt zugesetzt werden. Das vorgenannte gilt auch für die anderen Inhibitoren, falls das Inhibitorgemisch solche umfaßt. Selbstverständlich können die Destillations(Rektifikations)kolonnen bei vorgenannter Anwendung des erfindungsgemäßen Verfahrens von molekularem Sauerstoff oder einem Gemisch

desselben mit einem Inertgas, z.B. Luft, durchströmt werden.

Auch kann die Zugabe von Inhibitoren a) und Inhibitoren b) auch an unterschiedlichen Zugabeorten vorgenommen werden. So können z.B. die Inhibitoren b) am Kopf der Rektifikationskolonne und die Inhibitoren a) in den Sumpf und/oder den Zulauf der Rektifikationskolonne gegeben werden. Dies gilt sowohl für solche Rektifikationen im Rahmen derer der (Meth)acrylsäureester über Kopf-, über Sumpf- und/oder über Seitenabzug abgetrennt wird. Auch kann es zweckmäßig sein, das erfindungsgemäße Verfahren im Fall einer kontinuierlichen destillativen (rektifikativen) Abtrennung von (Meth)acrylsäureestern so durchzuführen, daß der erfindungsgemäß wenigstens eine zuzuführende Inhibitor b) nicht kontinuierlich, sondern lediglich von Zeit zu Zeit, d.h. periodisch wiederkehrend, zugegeben wird (z. B. am Kolonnenkopf, im Sumpf und/oder im Zulauf).

Alles über die erfindungsgemäße Stabilisierung bei der destillativen (rektifikativen) Abtrennung von (Meth) acrylsäureestern aus säurekatalysierten Veresterungsgemischen gesagte gilt in gleicher Weise auch bezüglich einer destillativen (rektifikativen) Abtrennung von (Meth)acrylsäure bzw. (Meth)acrolein aus diese enthaltenden Gemischen.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propan, Propen, tert.-Butanol, iso-Buten, iso-Butan, iso-Butyraldehyd oder Methacrolein erhältlich. Als Ausgangsverbindungen sind aber auch solche denkbar, aus welchen sich die eigentliche $C_3$-/$C_4$-Ausgangsverbindung während der Gasphasenoxidation erst intermediär bildet. Beispielhaft genannt sei der Methylether des tert.-Butanols.

Dabei werden diese Ausgangsgase, in der Regel mit Inertgasen wie Stickstoff, CO, $CO_2$, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt (vgl. z.B. DE-A 4 405 059, EP-A 253 409, EP-A 92 097, DE-A 44 31 957, DE-A 44 31 949, CN-A 11 053 52 und WO 97/36849).

Aufgrund zahlreicher im Verlauf der.katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine (Meth)acrylsäure sondern ein Reaktionsgasgemisch erhalten, das im wesentlichen (Meth) acrylsäure, die inerten Verdünnungsgase und Nebenprodukte enthält, aus welchem die (Meth)acrylsäure abgetrennt werden muß. Neben von (Meth)acrylsäure vergleichsweise einfach zu entfernenden und bei Folgeverwendungen der (Meth)acrylsäure weniger störenden Nebenprodukten wie z.B. Essigsäure, enthält das Reaktionsgasgemisch häufig auch mit (Meth)acrylsäure eng verwandte und daher von (Meth)acrylsäure schwer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie zusätzlich gegebenenfalls Maleinsäureanhydrid (bezogen auf die im Reaktionsgasgemisch enthaltene Menge an (Meth)acrylsäure beträgt die Gesamtmenge dieser, bei Folgeverwendungen häufig erheblich störenden, Nebenkomponenten in der Regel ≤ 2 Gew-%, meist ≥ 0,05 Gew.-%).

Die DE-A 44 36 243 betrifft ein Verfahren zur Abtrennung von (Meth)acrylsäure aus dem Reaktionsgasgemisch der katalytischen Gasphasenoxidation durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt, dem in der Absorptionskolonne in natürlicher weise erfolgenden Absorptionsprozeß einen Rektifikationsprozeß überlagert, indem man der Absorptionskolonne eine über ihre, aufgrund ihres Kontaktes mit der Umgebungstemperatur erfolgende, natürliche Energieabgabe hinausgehende Energiemenge entzieht, und aus dem (Meth)acrylsäure und das Absorptionsmittel (Absorbens) als Hauptbestandteile sowie niedere Aldehyde und gegebenenfalls Maleinsäureanhydrid als Nebenbestandteile enthaltenden Flüssigkeitsablauf der Absorptionskolonne (Absorbat) die (Meth)acrylsäure rektifikativ abtrennt. Die dabei erhältliche (Meth)acrylsäure wird als Roh-(Meth)acrylsäure bezeichnet. Sie weist in der Regel eine Reinheit > 98 Gew.-% auf.

Als hochsiedende inerte hydrophobe organische Flüssigkeit (Absorbens) faßt die DE-A 44 36 243 dabei alle diejenigen Flüssigkeiten zusammen, deren Siedetemperatur bei Normaldruck (1 atm) oberhalb der Siedetemperatur der (Meth)acrylsäure liegt und die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Dieser Begriffsinhalt gilt auch hier.

Aus der DE-PS 2 136 396 und der DE-A 43 08 087 ist ebenfalls bekannt, Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit abzutrennen. Das Verfahren wird im wesentlichen so durchgeführt, daß man das Reaktionsgasgemisch in einer konventionellen Absorptionskolonne zu der absteigenden Absorptionsflüssigkeit im Gegenstrom führt, danach in einer Desorptionskolonne aus dem im wesentlichen aus Acrylsäure, dem Absorptionsmittel und Nebenkomponenten zusammengesetzten Flüssigkeitsablauf der Absorptionskolonne durch Strippen (Abstreifen) mit Inertgas die einfach abtrennbaren leichtflüchtigen Nebenkomponenten weitgehend entfernt und anschließend den (Meth)acrylsäure und das Absorbens als Hauptbestandteile enthaltenden Flüssigkeitsablauf der Desorptionskolonne zur Abtrennung von Roh-Acrylsäure rektifikativ behandelt.

Die DE-A 2 235 326 betrifft ebenfalls das Problem der rektifikativen Abtrennung von (Meth)acrylsäure aus deren Gemisch mit höher als (Meth)acrylsäure siedenden organischen Lösungsmitteln, wobei in diesem Stand der Technik als in Betracht zu ziehende organische Lösungsmittel vor allem auch höhere Alkohole oder Ester dieser oder anderer Alkohole, insbesondere mit (Meth)acrylsäure, genannt werden.

Die DE-A 19810962 betrifft ebenfalls das Problem der rektifikativen Abtrennung von (Meth)acrylsäure aus deren Gemischen mit höher als (Meth)acrylsäure siedenden organischen Lösungsmitteln.

Das Problem einer rektifikativen Abtrennung von (Meth)acrylsäure erwächst aber auch dann, wenn man die (Meth)acrylsäure aus den Reaktionsgasen der katalytischen Gasphasenoxidation zunächst in Wasser aufnimmt und anschließend unter Zusatz eines organischen azeotropen Schleppers aus den wäßrigen (Meth)acrylsäure enthaltenden Gemischen das Wasser rektifikativ abtrennt.

Es besteht aber auch bei der rektifikativen Herstellung von Reinacrylsäure (Reinheit >99,7 Gew.-%) aus Roh-Acrylsäure (Reinheit >99 Gew.-%).

Bei allen diesen vorgenannten Rektifikationsproblemen ist die erfindungsgemäße Inhibierweise analog zur beschriebenen rektifikativen (Meth)acrylesterabtrennung anwendbar. Wird das hochsiedend hydrophobe organische Lösungsmittel, mit dem die (Meth)acrylsäure aus dem Reaktionsgemisch der Gasphasenoxidation extraktiv abgetrennt wird, nach der rektifikativen (Meth)acrylsäureabtrennung zur Extraktion rück-, d.h. im Kreis, geführt, kann es zweckmäßig sein, von Zeit zu Zeit das abgetrennte hochsiedende hydrophobe organische Lösungsmittel vor seiner Wiederverwendung zur Extraktion mit Inhibitor b) zu versetzen. Selbstverständlich kann das erfindungsgemäße Verfahren auch bei der Extraktion der (Meth)acrylsäure aus dem Reaktionsgemisch der Gasphasenoxidation selbst angewendet werden. Die erfindungsgemäße Stabilisierung empfiehlt sich auch für den Fall einer kristallisativen Abtrennung von (Meth)acrylsäure oder deren Ester enthaltenden Gemischen.

D.h., das erfindungsgemäße Inhibierverfahren ist grundsätzlich einsetzbar bei allen in der DE-A 19851983, DE-A 19851984, DE-A 19838783, DE-A 19838817, DE-A 19838845, DE-A 19836307, DE-A 1983795, DE-A 19837519, DE-A 19837520, DE-A 19837517, DE-A 19837518, DE-A 19832962 und DE-A 19833049 angesprochenen, (Meth) acrylsäure und/oder deren Ester betreffenden, Trennproblemen.

Es ist, wie bereits gesagt, auch anwendbar bei einer destillativen (rektifikativen) Abtrennung von (Meth)acrolein aus diese enthaltenden Gemischen. (Meth)acrolein ist in entsprechender Weise wie (Meth)acrylsäure z. B. durch katalytische Gasphasenoxidation erhältlich. Allerdings wird die Oxidation nach der ersten Oxidationsstufe nicht weitergeführt. Vielmehr wird das im Reaktionsgasgemisch enthaltene (Meth)acrolein in der Regel zunächst mittels Wasser extraktiv aus dem Reaktionsgasgemisch abgetrennt und anschließend destillativ (rektifikativ) aus der wäßrigen Lösung gewonnen. Für alle genannten Prozeßschritte eignet sich das erfindungsgemäße Stabilisierverfahren.

Erfindungsgemäß überrascht, daß Inhibitoren a) und Inhibitoren b) eine ausgeprägte synergistische Wirksamkeit im Hinblick auf die Stabilisierung von als Reinsubstanz oder als Bestandteil von Gemischen vorliegenden chemischen Verbindungen, die wenigstens eine ethylenisch ungesättigte Gruppe aufweisen, gegen unerwünschte radikalische Polymerisation zeigen. Dies gilt im wesentlichen unabhängig vom pH-Wert und sowohl für niedere (z.B. Raumtemperatur) als auch erhöhte Temperaturen, wie sie z.B. im Rahmen von thermischen physikalischen Trennverfahren als auch für bei erhöhten Temperaturen ablaufende chemische Umsetzungen üblich sind.

Insbesondere gilt das vorgenannte für eine Stabilisierung von (Meth)acrylsäure und/oder deren Ester, deren ethylenisch ungesättigte Doppelbindung bezüglich einer radikalischen Polymerisation besonders aktiv ist.

In der Regel werden die Inhibitoren a), b) erfindungsgemäß so gewählt, daß sie in der zu stabilisierenden Substanz in ihrer Einsatzmenge in vollem Umfang löslich sind. Häufig erfolgt ihre Zugabe nicht als Reinsubstanz, sondern als Suspension, Emulsion oder Lösung. Als Lösungs- und/oder Dispergiermedium kommen insbesondere diejenigen Substanzen in Betracht, die Bestandteil des zu stabilisierenden Systems sind. D.h., z.B. bei chemischen Umsetzungen wie Veresterungen alle Edukte und Produkte. Bei Extraktionen insbesondere das Extraktionsmedium oder eine Komponente davon.

Erfindungsgemäß besonders geeignete Kombinationen aus Inhibitoren a) und Inhibitoren b) sind:

| Inhibitor a) | Inhibitor b) |
|---|---|
| 4-Hydroxy-2,2,6,6-tetra-methyl-1-oxyl-piperidin | $P(OH)_3$ |
| 4-Hydroxy-2,2,6,6-tetra-methyl-1-oxyl-piperidin | $P(OMethyl)_3$ |
| 4-Hydroxy-2,2,6,6-tetra-methyl-1-oxyl-piperidin | $P(OEthyl)_3$ |
| 4-Hydroxy-2,2,6,6-tetra-methyl-1-oxyl-piperidin | $P(Oiso\text{-}Propyl)_3$ |
| 4-Hydroxy-2,2,6,6-tetra-methyl-1-oxyl-piperidin | $P(OPropyl)_3$ |
| 4-Hydroxy-2,2,6,6-tetra-methyl-1-oxyl-piperidin | $P(On\text{-}Butyl)_3$ |
| 4-Hydroxy-2,2,6,6-tetra-methyl-1-oxyl-piperidin | $P(Osec\text{-}Butyl)_3$ |
| 4-Hydroxy-2,2,6,6-tetra-methyl-1-oxyl-piperidin | $P(Otert.\text{-}Butyl)_3$ |
| 4-Hydroxy-2,2,6,6-tetra-methyl-1-oxyl-piperidin | $P(OPhenyl)_3$ |

| Inhibitor a) | Inhibitor b) |
|---|---|
| 4-Hydroxy-2,2,6,6-tetra-methyl-1-oxyl-piperidin | $P(OH)(OMethyl)_2$ |
| 4-Hydroxy-2,2,6,6-tetra-methyl-1-oxyl-piperidin | $P(OH)(OEthyl)_2$ |
| 4-Hydroxy-2,2,6,6-tetra-methyl-1-oxyl-piperidin | $P(OH)(Oiso\text{-}Propyl)_2$ |
| 4-Hydroxy-2,2,6,6-tetra-methyl-1-oxyl-piperidin | $P(OH)(OPropyl)_2$ |
| 4-Hydroxy-2,2,6,6-tetra-methyl-1-oxyl-piperidin | $P(OH)(On\text{-}Butyl)_2$ |
| 4-Hydroxy-2,2,6,6-tetra-methyl-1-oxyl-piperidin | $P(OH)(Osec\text{-}Butyl)_2$ |
| 4-Hydroxy-2,2,6,6-tetra-methyl-1-oxyl-piperidin | $P(OH)(Otert.\text{-}Butyl)_2$ |
| 4-Hydroxy-2,2,6,6-tetra-methyl-1-oxyl-piperidin | $P(OH)(OPhenyl)_2$ |
| 4-Hydroxy-2,2,6,6-tetra-methyl-1-oxyl-piperidin | $P(OH)_2(OMethyl)$ |
| 4-Hydroxy-2,2,6,6-tetra-methyl-1-oxyl-piperidin | $P(OH)_2(OEthyl)$ |
| 4-Hydroxy-2,2,6,6-tetra-methyl-1-oxyl-piperidin | $P(OH)_2(Oiso\text{-}Propyl)$ |
| 4-Hydroxy-2,2,6,6-tetra-methyl-1-oxyl-piperidin | $P(OH)_2(OPropyl)$ |
| 4-Hydroxy-2,2,6,6-tetra-methyl-1-oxyl-piperidin | $P(OH)_2(On\text{-}Butyl)$ |
| 4-Hydroxy-2,2,6,6-tetra-methyl-1-oxyl-piperidin | $P(OH)_2(Osec\text{-}Butyl)$ |
| 4-Hydroxy-2,2,6,6-tetra-methyl-1-oxyl-piperidin | $P(OH)_2(Otert.\text{-}Butyl)$ |
| 4-Hydroxy-2,2,6,6-tetra-methyl-1-oxyl-piperidin | $P(OH)_2(OPhenyl)$ |
| 4-Hydroxy-2,2,6,6-tetra-methyl-1-oxyl-piperidin | $HP(OH)_2$ |
| 4-Hydroxy-2,2,6,6-tetra-methyl-1-oxyl-piperidin | $(Methyl)P(OMethyl)_2$ |
| 4-Hydroxy-2,2,6,6-tetra-methyl-1-oxyl-piperidin | $(Ethyl)P(OEthyl)_2$ |
| 4-Hydroxy-2,2,6,6-tetra-methyl-1-oxyl-piperidin | $(iso\text{-}Propyl)P(Oiso\text{-}Propyl)_2$ |
| 4-Hydroxy-2,2,6,6-tetra-methyl-1-oxyl-piperidin | $(Propyl)P(OPropyl)_2$ |
| 4-Hydroxy-2,2,6,6-tetra-methyl-1-oxyl-piperidin | $(n\text{-}Butyl)P(On\text{-}Butyl)_2$ |
| 4-Hydroxy-2,2,6,6-tetra-methyl-1-oxyl-piperidin | $(sec\text{-}Butyl)P(Osec\text{-}Butyl)_2$ |

| Inhibitor a) | Inhibitor b) |
|---|---|
| 4-Hydroxy-2,2,6,6-tetramethyl-1-oxyl-piperidin | (tert.-Butyl)P(Otert.-Butyl)$_2$ |
| 4-Hydroxy-2,2,6,6-tetramethyl-1-oxyl-piperidin | (Phenyl)P(OPhenyl)$_2$ |
| 4-Hydroxy-2,2,6,6-tetramethyl-1-oxyl-piperidin | O=P(OH)$_3$ |
| 4-Hydroxy-2,2,6,6-tetramethyl-1-oxyl-piperidin | O=P(OMethyl)$_3$ |
| 4-Hydroxy-2,2,6,6-tetramethyl-1-oxyl-piperidin | O=P(OEthyl)$_3$ |
| 4-Hydroxy-2,2,6,6-tetramethyl-1-oxyl-piperidin | O=P(OPropyl)$_3$ |
| 4-Hydroxy-2,2,6,6-tetramethyl-1-oxyl-piperidin | O=P(Oiso-Propyl)$_3$ |
| 4-Hydroxy-2,2,6,6-tetramethyl-1-oxyl-piperidin | O=P(On-Butyl)$_3$ |
| 4-Hydroxy-2,2,6,6-tetramethyl-1-oxyl-piperidin | O=P(Osec-Butyl)$_3$ |
| 4-Hydroxy-2,2,6,6-tetramethyl-1-oxyl-piperidin | O=P(Otert.-Butyl)$_3$ |
| 4-Hydroxy-2,2,6,6-tetramethyl-1-oxyl-piperidin | O=P(OPhenyl)$_3$ |
| 4-Hydroxy-2,2,6,6-tetramethyl-1-oxyl-piperidin | O<br>‖<br>HO-P(OMethyl)$_2$ |
| 4-Hydroxy-2,2,6,6-tetramethyl-1-oxyl-piperidin | O<br>‖<br>HO-P(OEthyl)$_2$ |
| 4-Hydroxy-2,2,6,6-tetramethyl-1-oxyl-piperidin | O<br>‖<br>HO-P(Oiso-Propyl)$_2$ |
| 4-Hydroxy-2,2,6,6-tetramethyl-1-oxyl-piperidin | O<br>‖<br>HO-P(OPropyl)$_2$ |
| 4-Hydroxy-2,2,6,6-tetramethyl-1-oxyl-piperidin | O<br>‖<br>HO-P(On-Butyl)$_2$ |
| 4-Hydroxy-2,2,6,6-tetramethyl-1-oxyl-piperidin | O<br>‖<br>HO-P(Osec-Butyl)$_2$ |
| 4-Hydroxy-2,2,6,6-tetramethyl-1-oxyl-piperidin | O<br>‖<br>HO-P(Otert.-Butyl)$_2$ |
| 4-Hydroxy-2,2,6,6-tetramethyl-1-oxyl-piperidin | O<br>‖<br>HO-P(OPhenyl)$_2$ |
| 4-Hydroxy-2,2,6,6-tetramethyl-1-oxyl-piperidin | O=P(OH)$_2$(OMethyl) |

| Inhibitor a) | Inhibitor b) |
|---|---|
| 4-Hydroxy-2,2,6,6-tetra-methyl-1-oxyl-piperidin | $O=P(OH)_2$(OEthyl) |
| 4-Hydroxy-2,2,6,6-tetra-methyl-1-oxyl-piperidin | $O=P(OH)_2$(Oiso-Propyl) |
| 4-Hydroxy-2,2,6,6-tetra-methyl-1-oxyl-piperidin | $O=P(OH)_2$(OPropyl) |
| 4-Hydroxy-2,2,6,6-tetra-methyl-1-oxyl-piperidin | $O=P(OH)_2$(On-Butyl) |
| 4-Hydroxy-2,2,6,6-tetra-methyl-1-oxyl-piperidin | $O=P(OH)_2$(Osec.-Butyl) |
| 4-Hydroxy-2,2,6,6-tetra-methyl-1-oxyl-piperidin | $O=P(OH)_2$(Otert.-Butyl) |
| 4-Hydroxy-2,2,6,6-tetra-methyl-1-oxyl-piperidin | $O=P(OH)_2$(OPhenyl) |
| 4-Methoxy-2,2,6,6-tetra-methyl-1-oxyl-piperidin | $P(OH)_3$ |
| 4-Methoxy-2,2,6,6-tetra-methyl-1-oxyl-piperidin | $P(OMethyl)_3$ |
| 4-Methoxy-2,2,6,6-tetra-methyl-1-oxyl-piperidin | $P(OEthyl)_3$ |
| 4-Methoxy-2,2,6,6-tetra-methyl-1-oxyl-piperidin | P(Oiso-Propyl)$_3$ |
| 4-Methoxy-2,2,6,6-tetra-methyl-1-oxyl-piperidin | $P(OPropyl)_3$ |
| 4-Methoxy-2,2,6,6-tetra-methyl-1-oxyl-piperidin | P(On-Butyl)$_3$ |
| 4-Methoxy-2,2,6,6-tetra-methyl-1-oxyl-piperidin | P(Osec-Butyl)$_3$ |
| 4-Methoxy-2,2,6,6-tetra-methyl-1-oxyl-piperidin | P(Otert.-Butyl)$_3$ |
| 4-Methoxy-2,2,6,6-tetra-methyl-1-oxyl-piperidin | $P(OPhenyl)_3$ |
| 4-Methoxy-2,2,6,6-tetra-methyl-1-oxyl-piperidin | $P(OH)(OMethyl)_2$ |
| 4-Methoxy-2,2,6,6-tetra-methyl-1-oxyl-piperidin | $P(OH)(OEthyl)_2$ |
| 4-Methoxy-2,2,6,6-tetra-methyl-1-oxyl-piperidin | P(OH)(Oiso-Propyl)$_2$ |
| 4-Methoxy-2,2,6,6-tetra-methyl-1-oxyl-piperidin | $P(OH)(OPropyl)_2$ |
| 4-Methoxy-2,2,6,6-tetra-methyl-1-oxyl-piperidin | P(OH)(On-Butyl)$_2$ |
| 4-Methoxy-2,2,6,6-tetra-methyl-1-oxyl-piperidin | P(OH)(Osec-Butyl)$_2$ |
| 4-Methoxy-2,2,6,6-tetra-methyl-1-oxyl-piperidin | P(OH)(Otert.-Butyl)$_2$ |

| Inhibitor a) | Inhibitor b) |
|---|---|
| 4-Methoxy-2,2,6,6-tetra-methyl-1-oxyl-piperidin | $P(OH)(OPhenyl)_2$ |
| 4-Methoxy-2,2,6,6-tetra-methyl-1-oxyl-piperidin | $P(OH)_2(OMethyl)$ |
| 4-Methoxy-2,2,6,6-tetra-methyl-1-oxyl-piperidin | $P(OH)_2(OEthyl)$ |
| 4-Methoxy-2,2,6,6-tetra-methyl-1-oxyl-piperidin | $P(OH)_2$(Oiso-Propyl) |
| 4-Methoxy-2,2,6,6-tetra-methyl-1-oxyl-piperidin | $P(OH)_2(OPropyl)$ |
| 4-Methoxy-2,2,6,6-tetra-methyl-1-oxyl-piperidin | $P(OH)_2$(On-Butyl) |
| 4-Methoxy-2,2,6,6-tetra-methyl-1-oxyl-piperidin | $P(OH)_2$(Osec-Butyl) |
| 4-Methoxy-2,2,6,6-tetra-methyl-1-oxyl-piperidin | $P(OH)_2$(Otert.-Butyl) |
| 4-Methoxy-2,2,6,6-tetra-methyl-1-oxyl-piperidin | $P(OH)_2(OPhenyl)$ |
| 4-Methoxy-2,2,6,6-tetra-methyl-1-oxyl-piperidin | $HP(OH)_2$ |
| 4-Methoxy-2,2,6,6-tetra-methyl-1-oxyl-piperidin | (Methyl)P$(OMethyl)_2$ |
| 4-Methoxy-2,2,6,6-tetra-methyl-1-oxyl-piperidin | (Ethyl)P$(OEthyl)_2$ |
| 4-Methoxy-2,2,6,6-tetra-methyl-1-oxyl-piperidin | (iso-Propyl)P(Oiso-Propyl)$_2$ |
| 4-Methoxy-2,2,6,6-tetra-methyl-1-oxyl-piperidin | (Propyl)P$(OPropyl)_2$ |
| 4-Methoxy-2,2,6,6-tetra-methyl-1-oxyl-piperidin | (n-Butyl)P(On-Butyl)$_2$ |
| 4-Methoxy-2,2,6,6-tetra-methyl-1-oxyl-piperidin | (sec-Butyl)P(Osec-Butyl)$_2$ |
| 4-Methoxy-2,2,6,6-tetra-methyl-1-oxyl-piperidin | (tert.-Butyl)P(Otert.-Butyl)$_2$ |
| 4-Methoxy-2,2,6,6-tetra-methyl-1-oxyl-piperidin | (Phenyl)P$(OPhenyl)_2$ |
| 4-Methoxy-2,2,6,6-tetra-methyl-1-oxyl-piperidin | $O=P(OH)_3$ |
| 4-Methoxy-2,2,6,6-tetra-methyl-1-oxyl-piperidin | $O=P(OMethyl)_3$ |
| 4-Methoxy-2,2,6,6-tetra-methyl-1-oxyl-piperidin | $O=P(OEthyl)_3$ |
| 4-Methoxy-2,2,6,6-tetra-methyl-1-oxyl-piperidin | $O=P(OPropyl)_3$ |
| 4-Methoxy-2,2,6,6-tetra-methyl-1-oxyl-piperidin | O=P(Oiso-Propyl)$_3$ |

| Inhibitor a) | Inhibitor b) |
|---|---|
| 4-Methoxy-2,2,6,6-tetra-methyl-1-oxyl-piperidin | O=P(On-Butyl)$_3$ |
| 4-Methoxy-2,2,6,6-tetra-methyl-1-oxyl-piperidin | O=P(Osec-Butyl)$_3$ |
| 4-Methoxy-2,2,6,6-tetra-methyl-1-oxyl-piperidin | O=P(Otert.-Butyl)$_3$ |
| 4-Methoxy-2,2,6,6-tetra-methyl-1-oxyl-piperidin | O=P(OPhenyl)$_3$ |
| 4-Methoxy-2,2,6,6-tetra-methyl-1-oxyl-piperidin | O<br>‖<br>HO-P(OMethyl)$_2$ |
| 4-Methoxy-2,2,6,6-tetra-methyl-1-oxyl-piperidin | O<br>‖<br>HO-P(OEthyl)$_2$ |
| 4-Methoxy-2,2,6,6-tetra-methyl-1-oxyl-piperidin | O<br>‖<br>HO-P(Oiso-Propyl)$_2$ |
| 4-Methoxy-2,2,6,6-tetra-methyl-1-oxyl-piperidin | O<br>‖<br>HO-P(OPropyl)$_2$ |
| 4-Methoxy-2,2,6,6-tetra-methyl-1-oxyl-piperidin | O<br>‖<br>HO-P(On-Butyl)$_2$ |
| 4-Methoxy-2,2,6,6-tetra-methyl-1-oxyl-piperidin | O<br>‖<br>HO-P(Osec-Butyl)$_2$ |
| 4-Methoxy-2,2,6,6-tetra-methyl-1-oxyl-piperidin | O<br>‖<br>HO-P(Otert.-Butyl)$_2$ |
| 4-Methoxy-2,2,6,6-tetra-methyl-1-oxyl-piperidin | O<br>‖<br>HO-P(OPhenyl)$_2$ |
| 4-Methoxy-2,2,6,6-tetra-methyl-1-oxyl-piperidin | O=P(OH)$_2$(OMethyl) |
| 4-Methoxy-2,2,6,6-tetra-methyl-1-oxyl-piperidin | O=P(OH)$_2$(OEthyl) |
| 4-Methoxy-2,2,6,6-tetra-methyl-1-oxyl-piperidin | O=P(OH)$_2$(Oiso-Propyl) |
| 4-Methoxy-2,2,6,6-tetra-methyl-1-oxyl-piperidin | O=P(OH)$_2$(OPropyl) |
| 4-Methoxy-2,2,6,6-tetra-methyl-1-oxyl-piperidin | O=P(OH)$_2$(On-Butyl) |
| 4-Methoxy-2,2,6,6-tetra-methyl-1-oxyl-piperidin | O=P(OH)$_2$(Osec.-Butyl) |
| 4-Methoxy-2,2,6,6-tetra-methyl-1-oxyl-piperidin | O=P(OH)$_2$(Otert.-Butyl) |
| 4-Methoxy-2,2,6,6-tetra-methyl-1-oxyl-piperidin | O=P(OH)$_2$(OPhenyl) |

| Inhibitor a) | Inhibitor b) |
|---|---|
| 4-Oxo-2,2,6,6-tetra-methyl-1-oxyl-piperidin | $P(OH)_3$ |
| 4-Oxo-2,2,6,6-tetra-methyl-1-oxyl-piperidin | $P(OMethyl)_3$ |
| 4-Oxo-2,2,6,6-tetra-methyl-1-oxyl-piperidin | $P(OEthyl)_3$ |
| 4-Oxo-2,2,6,6-tetra-methyl-1-oxyl-piperidin | $P(Oiso\text{-}Propyl)_3$ |
| 4-Oxo-2,2,6,6-tetra-methyl-1-oxyl-piperidin | $P(OPropyl)_3$ |
| 4-Oxo-2,2,6,6-tetra-methyl-1-oxyl-piperidin | $P(On\text{-}Butyl)_3$ |
| 4-Oxo-2,2,6,6-tetra-methyl-1-oxyl-piperidin | $P(Osec\text{-}Butyl)_3$ |
| 4-Oxo-2,2,6,6-tetra-methyl-1-oxyl-piperidin | $P(Otert.\text{-}Butyl)_3$ |
| 4-Oxo-2,2,6,6-tetra-methyl-1-oxyl-piperidin | $P(OPhenyl)_3$ |
| 4-Oxo-2,2,6,6-tetra-methyl-1-oxyl-piperidin | $P(OH)(OMethyl)_2$ |
| 4-Oxo-2,2,6,6-tetra-methyl-1-oxyl-piperidin | $P(OH)(OEthyl)_2$ |
| 4-Oxo-2,2,6,6-tetra-methyl-1-oxyl-piperidin | $P(OH)(Oiso\text{-}Propyl)_2$ |
| 4-Oxo-2,2,6,6-tetra-methyl-1-oxyl-piperidin | $P(OH)(OPropyl)_2$ |
| 4-Oxo-2,2,6,6-tetra-methyl-1-oxyl-piperidin | $P(OH)(On\text{-}Butyl)_2$ |
| 4-Oxo-2,2,6,6-tetra-methyl-1-oxyl-piperidin | $P(OH)(Osec\text{-}Butyl)_2$ |
| 4-Oxo-2,2,6,6-tetra-methyl-1-oxyl-piperidin | $P(OH)(Otert.\text{-}Butyl)_2$ |
| 4-Oxo-2,2,6,6-tetra-methyl-1-oxyl-piperidin | $P(OH)(OPhenyl)_2$ |
| 4-Oxo-2,2,6,6-tetra-methyl-1-oxyl-piperidin | $P(OH)_2(OMethyl)$ |
| 4-Oxo-2,2,6,6-tetra-methyl-1-oxyl-piperidin | $P(OH)_2(OEthyl)$ |
| 4-Oxo-2,2,6,6-tetra-methyl-1-oxyl-piperidin | $P(OH)_2(Oiso\text{-}Propyl)$ |
| 4-Oxo-2,2,6,6-tetra-methyl-1-oxyl-piperidin | $P(OH)_2(OPropyl)$ |
| 4-Oxo-2,2,6,6-tetra-methyl-1-oxyl-piperidin | $P(OH)_2(On\text{-}Butyl)$ |
| 4-Oxo-2,2,6,6-tetra-methyl-1-oxyl-piperidin | $P(OH)_2(Osec\text{-}Butyl)$ |

| Inhibitor a) | Inhibitor b) |
|---|---|
| 4-Oxo-2,2,6,6-tetra-methyl-1-oxyl-piperidin | P(OH)$_2$(Otert.-Butyl) |
| 4-Oxo-2,2,6,6-tetra-methyl-1-oxyl-piperidin | P(OH)$_2$(OPhenyl) |
| 4-Oxo-2,2,6,6-tetra-methyl-1-oxyl-piperidin | HP(OH)$_2$ |
| 4-Oxo-2,2,6,6-tetra-methyl-1-oxyl-piperidin | (Methyl)P(OMethyl)$_2$ |
| 4-Oxo-2,2,6,6-tetra-methyl-1-oxyl-piperidin | (Ethyl)P(OEthyl)$_2$ |
| 4-Oxo-2,2,6,6-tetra-methyl-1-oxyl-piperidin | (iso-Propyl)P(Oiso-Propyl)$_2$ |
| 4-Oxo-2,2,6,6-tetra-methyl-1-oxyl-piperidin | (Propyl)P(OPropyl)$_2$ |
| 4-Oxo-2,2,6,6-tetra-methyl-1-oxyl-piperidin | (n-Butyl)P(On-Butyl)$_2$ |
| 4-Oxo-2,2,6,6-tetra-methyl-1-oxyl-piperidin | (sec-Butyl)P(Osec-Butyl)$_2$ |
| 4-Oxo-2,2,6,6-tetra-methyl-1-oxyl-piperidin | (tert.-Butyl)P(Otert.-Butyl)$_2$ |
| 4-Oxo-2,2,6,6-tetra-methyl-1-oxyl-piperidin | (Phenyl)P(OPhenyl)$_2$ |
| 4-Oxo-2,2,6,6-tetra-methyl-1-oxyl-piperidin | O=P(OH)$_3$ |
| 4-Oxo-2,2,6,6-tetra-methyl-1-oxyl-piperidin | O=P(OMethyl)$_3$ |
| 4-Oxo-2,2,6,6-tetra-methyl-1-oxyl-piperidin | O=P(OEthyl)$_3$ |
| 4-Oxo-2,2,6,6-tetra-methyl-1-oxyl-piperidin | O=P(OPropyl)$_3$ |
| 4-Oxo-2,2,6,6-tetra-methyl-1-oxyl-piperidin | O=P(Oiso-Propyl)$_3$ |
| 4-Oxo-2,2,6,6-tetra-methyl-1-oxyl-piperidin | O=P(On-Butyl)$_3$ |
| 4-Oxo-2,2,6,6-tetra-methyl-1-oxyl-piperidin | O=P(Osec-Butyl)$_3$ |
| 4-Oxo-2,2,6,6-tetra-methyl-1-oxyl-piperidin | O=P(Otert.-Butyl)$_3$ |
| 4-Oxo-2,2,6,6-tetra-methyl-1-oxyl-piperidin | O=P(OPhenyl)$_3$ |
| 4-Oxo-2,2,6,6-tetra-methyl-1-oxyl-piperidin | O<br>‖<br>HO-P(OMethyl)$_2$ |
| 4-Oxo-2,2,6,6-tetra-methyl-1-oxyl-piperidin | O<br>‖<br>HO-P(OEthyl)$_2$ |

| Inhibitor a) | Inhibitor b) |
|---|---|
| 4-Oxo-2,2,6,6-tetra-methyl-1-oxyl-piperidin | O<br>‖<br>HO-P(Oiso-Propyl)$_2$ |
| 4-Oxo-2,2,6,6-tetra-methyl-1-oxyl-piperidin | O<br>‖<br>HO-P(OPropyl)$_2$ |
| 4-Oxo-2,2,6,6-tetra-methyl-1-oxyl-piperidin | O<br>‖<br>HO-P(On-Butyl)$_2$ |
| 4-Oxo-2,2,6,6-tetra-methyl-1-oxyl-piperidin | O<br>‖<br>HO-P(Osec-Butyl)$_2$ |
| 4-Oxo-2,2,6,6-tetra-methyl-1-oxyl-piperidin | O<br>‖<br>HO-P(Otert.-Butyl)$_2$ |
| 4-Oxo-2,2,6,6-tetra-methyl-1-oxyl-piperidin | O<br>‖<br>HO-P(OPhenyl)$_2$ |
| 4-Oxo-2,2,6,6-tetra-methyl-1-oxyl-piperidin | O=P(OH)$_2$(OMethyl) |
| 4-Oxo-2,2,6,6-tetra-methyl-1-oxyl-piperidin | O=P(OH)$_2$(OEthyl) |
| 4-Oxo-2,2,6,6-tetra-methyl-1-oxyl-piperidin | O=P(OH)$_2$(Oiso-Propyl) |
| 4-Oxo-2,2,6,6-tetra-methyl-1-oxyl-piperidin | O=P(OH)$_2$(OPropyl) |
| 4-Oxo-2,2,6,6-tetra-methyl-1-oxyl-piperidin | O=P(OH)$_2$(On-Butyl) |
| 4-Oxo-2,2,6,6-tetra-methyl-1-oxyl-piperidin | O=P(OH)$_2$(Osec.-Butyl) |
| 4-Oxo-2,2,6,6-tetra-methyl-1-oxyl-piperidin | O=P(OH)$_2$(Otert.-Butyl) |
| 4-Oxo-2,2,6,6-tetra-methyl-1-oxyl-piperidin | O=P(OH)$_2$(OPhenyl) |
| N,N'-Bis(1-Oxyl-2,2,6,6-tetra-methyl-piperidin-4-yl)-N,N'-bis-formyl-1,6-diaminohexan | P(OH)$_3$ |
| N,N'-Bis(1-Oxyl-2,2,6,6-tetra-methyl-piperidin-4-yl)-N,N'-bis-formyl-1,6-diaminohexan | P(OMethyl)$_3$ |
| N,N'-Bis(1-Oxyl-2,2,6,6-tetra-methyl-piperidin-4-yl)-N,N'-bis-formyl-1,6-diaminohexan | P(OEthyl)$_3$ |
| N,N'-Bis(1-Oxyl-2,2,6,6-tetra-methyl-piperidin-4-yl)-N,N'-bis-formyl-1,6-diaminohexan | P(Oiso-Propyl)$_3$ |

| Inhibitor a) | Inhibitor b) |
|---|---|
| N,N'-Bis(1-Oxyl-2,2,6,6-tetra-methyl-piperidin-4-yl)-N,N'-bis-formyl-1,6-diaminohexan | $P(OPropyl)_3$ |
| N,N'-Bis(1-Oxyl-2,2,6,6-tetra-methyl-piperidin-4-yl)-N,N'-bis-formyl-1,6-diaminohexan | $P(On\text{-}Butyl)_3$ |
| N,N'-Bis(1-Oxyl-2,2,6,6-tetra-methyl-piperidin-4-yl)-N,N'-bis-formyl-1,6-diaminohexan | $P(Osec\text{-}Butyl)_3$ |
| N,N'-Bis(1-Oxyl-2,2,6,6-tetra-methyl-piperidin-4-yl)-N,N'-bis-formyl-1,6-diaminohexan | $P(Otert.\text{-}Butyl)_3$ |
| N,N'-Bis(1-Oxyl-2,2,6,6-tetra-methyl-piperidin-4-yl)-N,N'-bis-formyl-1,6-diaminohexan | $P(OPhenyl)_3$ |
| N,N'-Bis(1-Oxyl-2,2,6,6-tetra-methyl-piperidin-4-yl)-N,N'-bis-formyl-1,6-diaminohexan | $P(OH)(OMethyl)_2$ |
| N,N'-Bis(1-Oxyl-2,2,6,6-tetra-methyl-piperidin-4-yl)-N,N'-bis-formyl-1,6-diaminohexan | $P(OH)(OEthyl)_2$ |
| N,N'-Bis(1-Oxyl-2,2,6,6-tetra-methyl-piperidin-4-yl)-N,N'-bis-formyl-1,6-diaminohexan | $P(OH)(Oiso\text{-}Propyl)_2$ |
| N,N'-Bis(1-Oxyl-2,2,6,6-tetra-methyl-piperidin-4-yl)-N,N'-bis-formyl-1,6-diaminohexan | $P(OH)(OPropyl)_2$ |
| N,N'-Bis(1-Oxyl-2,2,6,6-tetra-methyl-piperidin-4-yl)-N,N'-bis-formyl-1,6-diaminohexan | $P(OH)(On\text{-}Butyl)_2$ |
| N,N'-Bis(1-Oxyl-2,2,6,6-tetra-methyl-piperidin-4-yl)-N,N'-bis-formyl-1,6-diaminohexan | $P(OH)(Osec\text{-}Butyl)_2$ |
| N,N'-Bis(1-Oxyl-2,2,6,6-tetra-methyl-piperidin-4-yl)-N,N'-bis-formyl-1,6-diaminohexan | $P(OH)(Otert.\text{-}Butyl)_2$ |
| N,N'-Bis(1-Oxyl-2,2,6,6-tetra-methyl-piperidin-4-yl)-N,N'-bis-formyl-1,6-diaminohexan | $P(OH)(OPhenyl)_2$ |
| N,N'-Bis(1-Oxyl-2,2,6,6-tetra-methyl-piperidin-4-yl)-N,N'-bis-formyl-1,6-diaminohexan | $P(OH)_2(OMethyl)$ |
| N,N'-Bis(1-Oxyl-2,2,6,6-tetra-methyl-piperidin-4-yl)-N,N'-bis-formyl-1,6-diaminohexan | $P(OH)_2(OEthyl)$ |
| N,N'-Bis(1-Oxyl-2,2,6,6-tetra-methyl-piperidin-4-yl)-N,N'-bis-formyl-1,6-diaminohexan | $P(OH)_2(Oiso\text{-}Propyl)$ |

| Inhibitor a) | Inhibitor b) |
|---|---|
| N,N'-Bis(1-Oxyl-2,2,6,6-tetramethylpiperidin-4-yl)-N,N'-bis-formyl-1,6-diaminohexan | $P(OH)_2$(OPropyl) |
| N,N'-Bis(1-Oxyl-2,2,6,6-tetramethylpiperidin-4-yl)-N,N'-bis-formyl-1,6-diaminohexan | $P(OH)_2$(On-Butyl) |
| N,N'-Bis(1-Oxyl-2,2,6,6-tetramethylpiperidin-4-yl)-N,N'-bis-formyl-1,6-diaminohexan | $P(OH)_2$(Osec-Butyl) |
| N,N'-Bis(1-Oxyl-2,2,6,6-tetramethylpiperidin-4-yl)-N,N'-bis-formyl-1,6-diaminohexan | $P(OH)_2$(Otert.-Butyl) |
| N,N'-Bis(1-Oxyl-2,2,6,6-tetramethylpiperidin-4-yl)-N,N'-bis-formyl-1,6-diaminohexan | $P(OH)_2$(OPhenyl) |
| N,N'-Bis(1-Oxyl-2,2,6,6-tetramethylpiperidin-4-yl)-N,N'-bis-formyl-1,6-diaminohexan | $HP(OH)_2$ |
| N,N'-Bis(1-Oxyl-2,2,6,6-tetramethylpiperidin-4-yl)-N,N'-bis-formyl-1,6-diaminohexan | (Methyl)P(OMethyl)$_2$ |
| N,N'-Bis(1-Oxyl-2,2,6,6-tetramethylpiperidin-4-yl)-N,N'-bis-formyl-1,6-diaminohexan | (Ethyl)P(OEthyl)$_2$ |
| N,N'-Bis(1-Oxyl-2,2,6,6-tetramethylpiperidin-4-yl)-N,N'-bis-formyl-1,6-diaminohexan | (iso-Propyl)P(Oiso-Propyl)$_2$ |
| N,N'-Bis(1-Oxyl-2,2,6,6-tetramethylpiperidin-4-yl)-N,N'-bis-formyl-1,6-diaminohexan | (Propyl)P(OPropyl)$_2$ |
| N,N'-Bis(1-Oxyl-2,2,6,6-tetramethylpiperidin-4-yl)-N,N'-bis-formyl-1,6-diaminohexan | (n-Butyl)P(On-Butyl)$_2$ |
| N,N'-Bis(1-Oxyl-2,2,6,6-tetramethylpiperidin-4-yl)-N,N'-bis-formyl-1,6-diaminohexan | (sec-Butyl)P(Osec-Butyl)$_2$ |
| N,N'-Bis(1-Oxyl-2,2,6,6-tetramethylpiperidin-4-yl)-N,N'-bis-formyl-1,6-diaminohexan | (tert.-Butyl)P(Otert.-Butyl)$_2$ |
| N,N'-Bis(1-Oxyl-2,2,6,6-tetramethylpiperidin-4-yl)-N,N'-bis-formyl-1,6-diaminohexan | (Phenyl)P(OPhenyl)$_2$ |
| N,N'-Bis(1-Oxyl-2,2,6,6-tetramethylpiperidin-4-yl)-N,N'-bis-formyl-1,6-diaminohexan | $O=P(OH)_3$ |
| N,N'-Bis(1-Oxyl-2,2,6,6-tetramethylpiperidin-4-yl)-N,N'-bis-formyl-1,6-diaminohexan | O=P(OMethyl)$_3$ |

| Inhibitor a) | Inhibitor b) |
|---|---|
| N,N'-Bis(1-Oxyl-2,2,6,6-tetra-methyl-piperidin-4-yl)-N,N'-bis-formyl-1,6-diaminohexan | O=P(OEthyl)$_3$ |
| N,N'-Bis(1-Oxyl-2,2,6,6-tetra-methyl-piperidin-4-yl)-N,N'-bis-formyl-1,6-diaminohexan | O=P(OPropyl)$_3$ |
| N,N'-Bis(1-Oxyl-2,2,6,6-tetra-methyl-piperidin-4-yl)-N,N'-bis-formyl-1,6-diaminohexan | O=P(Oiso-Propyl)$_3$ |
| N,N'-Bis(1-Oxyl-2,2,6,6-tetra-methyl-piperidin-4-yl)-N,N'-bis-formyl-1,6-diaminohexan | O=P(On-Butyl)$_3$ |
| N,N'-Bis(1-Oxyl-2,2,6,6-tetra-methyl-piperidin-4-yl)-N,N'-bis-formyl-1,6-diaminohexan | O=P(Osec-Butyl)$_3$ |
| N,N'-Bis(1-Oxyl-2,2,6,6-tetra-methyl-piperidin-4-yl)-N,N'-bis-formyl-1,6-diaminohexan | O=P(Otert.-Butyl)$_3$ |
| N,N'-Bis(1-Oxyl-2,2,6,6-tetra-methyl-piperidin-4-yl)-N,N'-bis-formyl-1,6-diaminohexan | O=P(OPhenyl)$_3$ |
| N,N'-Bis(1-Oxyl-2,2,6,6-tetra-methyl-piperidin-4-yl)-N,N'-bis-formyl-1,6-diaminohexan | O<br>‖<br>HO-P(OMethyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(OEthyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(Oiso-Propyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(OPropyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(On-Butyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(Osec-Butyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(Otert.-Butyl)$_2$ |
| N,N'-Bis(1-Oxyl-2,2,6,6-tetra-methyl-piperidin-4-yl)-N,N'-bis-formyl-1,6-diaminohexan | O<br>‖<br>HO-P(OPhenyl)$_2$ |
| N,N'-Bis(1-Oxyl-2,2,6,6-tetra-methyl-piperidin-4-yl)-N,N'-bis-formyl-1,6-diaminohexan | O=P(OH)$_2$(OMethyl) |

| Inhibitor a) | Inhibitor b) |
|---|---|
| N,N'-Bis(1-Oxyl-2,2,6,6-tetra-methyl-piperidin-4-yl)-N,N'-bis-formyl-1,6-diaminohexan | $O=P(OH)_2$(OEthyl) |
| N,N'-Bis(1-Oxyl-2,2,6,6-tetra-methyl-piperidin-4-yl)-N,N'-bis-formyl-1,6-diaminohexan | $O=P(OH)_2$(Oiso-Propyl) |
| N,N'-Bis(1-Oxyl-2,2,6,6-tetra-methyl-piperidin-4-yl)-N,N'-bis-formyl-1,6-diaminohexan | $O=P(OH)_2$(OPropyl) |
| N,N'-Bis(1-Oxyl-2,2,6,6-tetra-methyl-piperidin-4-yl)-N,N'-bis-formyl-1,6-diaminohexan | $O=P(OH)_2$(On-Butyl) |
| N,N'-Bis(1-Oxyl-2,2,6,6-tetra-methyl-piperidin-4-yl)-N,N'-bis-formyl-1,6-diaminohexan | $O=P(OH)_2$(Osec.-Butyl) |
| N,N'-Bis(1-Oxyl-2,2,6,6-tetra-methyl-piperidin-4-yl)-N,N'-bis-formyl-1,6-diaminohexan | $O=P(OH)_2$(Otert.-Butyl) |
| N,N'-Bis(1-Oxyl-2,2,6,6-tetra-methyl-piperidin-4-yl)-N,N'-bis-formyl-1,6-diaminohexan | $O=P(OH)_2$(OPhenyl) |
| Bis(1-Oxy-2,2,6,6-tetra-methyl)-piperidin-4-yl)-sebacat | $P(OH)_3$ |
| Bis(1-Oxy-2,2,6,6-tetra-methyl)-piperidin-4-yl)-sebacat | $P(OMethyl)_3$ |
| Bis(1-Oxy-2,2,6,6-tetra-methyl)-piperidin-4-yl)-sebacat | $P(OEthyl)_3$ |
| Bis(1-Oxy-2,2,6,6-tetra-methyl)-piperidin-4-yl)-sebacat | $P(Oiso\text{-}Propyl)_3$ |
| Bis(1-Oxy-2,2,6,6-tetra-methyl)-piperidin-4-yl)-sebacat | $P(OPropyl)_3$ |
| Bis(1-Oxy-2,2,6,6-tetra-methyl)-piperidin-4-yl)-sebacat | $P(On\text{-}Butyl)_3$ |
| Bis(1-Oxy-2,2,6,6-tetra-methyl)-piperidin-4-yl)-sebacat | $P(Osec\text{-}Butyl)_3$ |
| Bis(1-Oxy-2,2,6,6-tetra-methyl)-piperidin-4-yl)-sebacat | $P(Otert.\text{-}Butyl)_3$ |
| Bis(1-Oxy-2,2,6,6-tetra-methyl)-piperidin-4-yl)-sebacat | $P(OPhenyl)_3$ |
| Bis(1-Oxy-2,2,6,6-tetra-methyl)-piperidin-4-yl)-sebacat | $P(OH)(OMethyl)_2$ |
| Bis(1-Oxy-2,2,6,6-tetra-methyl)-piperidin-4-yl)-sebacat | $P(OH)(OEthyl)_2$ |
| Bis(1-Oxy-2,2,6,6-tetra-methyl)-piperidin-4-yl)-sebacat | $P(OH)(Oiso\text{-}Propyl)_2$ |
| Bis(1-Oxy-2,2,6,6-tetra-methyl)-piperidin-4-yl)-sebacat | $P(OH)(OPropyl)_2$ |

| Inhibitor a) | Inhibitor b) |
|---|---|
| Bis(1-Oxy-2,2,6,6-tetra-methyl)-piperidin-4-yl)-sebacat | $P(OH)(On\text{-}Butyl)_2$ |
| Bis(1-Oxy-2,2,6,6-tetra-methyl)-piperidin-4-yl)-sebacat | $P(OH)(Osec\text{-}Butyl)_2$ |
| Bis(1-Oxy-2,2,6,6-tetra-methyl)-piperidin-4-yl)-sebacat | $P(OH)(Otert.\text{-}Butyl)_2$ |
| Bis(1-Oxy-2,2,6,6-tetra-methyl)-piperidin-4-yl)-sebacat | $P(OH)(OPhenyl)_2$ |
| Bis(1-Oxy-2,2,6,6-tetra-methyl)-piperidin-4-yl)-sebacat | $P(OH)_2(OMethyl)$ |
| Bis(1-Oxy-2,2,6,6-tetra-methyl)-piperidin-4-yl)-sebacat | $P(OH)_2(OEthyl)$ |
| Bis(1-Oxy-2,2,6,6-tetra-methyl)-piperidin-4-yl)-sebacat | $P(OH)_2(Oiso\text{-}Propyl)$ |
| Bis(1-Oxy-2,2,6,6-tetra-methyl)-piperidin-4-yl)-sebacat | $P(OH)_2(OPropyl)$ |
| Bis(1-Oxy-2,2,6,6-tetra-methyl)-piperidin-4-yl)-sebacat | $P(OH)_2(On\text{-}Butyl)$ |
| Bis(1-Oxy-2,2,6,6-tetra-methyl)-piperidin-4-yl)-sebacat | $P(OH)_2(Osec\text{-}Butyl)$ |
| Bis(1-Oxy-2,2,6,6-tetra-methyl)-piperidin-4-yl)-sebacat | $P(OH)_2(Otert.\text{-}Butyl)$ |
| Bis(1-Oxy-2,2,6,6-tetra-methyl)-piperidin-4-yl)-sebacat | $P(OH)_2(OPhenyl)$ |
| Bis(1-Oxy-2,2,6,6-tetra-methyl)-piperidin-4-yl)-sebacat | $HP(OH)_2$ |
| Bis(1-Oxy-2,2,6,6-tetra-methyl)-piperidin-4-yl)-sebacat | $(Methyl)P(OMethyl)_2$ |
| Bis(1-Oxy-2,2,6,6-tetra-methyl)-piperidin-4-yl)-sebacat | $(Ethyl)P(OEthyl)_2$ |
| Bis(1-Oxy-2,2,6,6-tetra-methyl)-piperidin-4-yl)-sebacat | $(iso\text{-}Propyl)P(Oiso\text{-}Propyl)_2$ |
| Bis(1-Oxy-2,2,6,6-tetra-methyl)-piperidin-4-yl)-sebacat | $(Propyl)P(OPropyl)_2$ |
| Bis(1-Oxy-2,2,6,6-tetra-methyl)-piperidin-4-yl)-sebacat | $(n\text{-}Butyl)P(On\text{-}Butyl)_2$ |
| Bis(1-Oxy-2,2,6,6-tetra-methyl)-piperidin-4-yl)-sebacat | $(sec\text{-}Butyl)P(Osec\text{-}Butyl)_2$ |
| Bis(1-Oxy-2,2,6,6-tetra-methyl)-piperidin-4-yl)-sebacat | $(tert.\text{-}Butyl)P(Otert.\text{-}Butyl)_2$ |
| Bis(1-Oxy-2,2,6,6-tetra-methyl)-piperidin-4-yl)-sebacat | $(Phenyl)P(OPhenyl)_2$ |
| Bis(1-Oxy-2,2,6,6-tetra-methyl)-piperidin-4-yl)-sebacat | $O=P(OH)_3$ |
| Bis(1-Oxy-2,2,6,6-tetra-methyl)-piperidin-4-yl)-sebacat | $O=P(OMethyl)_3$ |

| Inhibitor a) | Inhibitor b) |
|---|---|
| Bis(1-Oxy-2,2,6,6-tetra-methyl)-piperidin-4-yl)-sebacat | O=P(OEthyl)$_3$ |
| Bis(1-Oxy-2,2,6,6-tetra-methyl)-piperidin-4-yl)-sebacat | O=P(OPropyl)$_3$ |
| Bis(1-Oxy-2,2,6,6-tetra-methyl)-piperidin-4-yl)-sebacat | O=P(Oiso-Propyl)$_3$ |
| Bis(1-Oxy-2,2,6,6-tetra-methyl)-piperidin-4-yl)-sebacat | O=P(On-Butyl)$_3$ |
| Bis(1-Oxy-2,2,6,6-tetra-methyl)-piperidin-4-yl)-sebacat | O=P(Osec-Butyl)$_3$ |
| Bis(1-Oxy-2,2,6,6-tetra-methyl)-piperidin-4-yl)-sebacat | O=P(Otert.-Butyl)$_3$ |
| Bis(1-Oxy-2,2,6,6-tetra-methyl)-piperidin-4-yl)-sebacat | O=P(OPhenyl)$_3$ |
| Bis(1-Oxy-2,2,6,6-tetra-methyl)-piperidin-4-yl)-sebacat | O<br>‖<br>HO-P(OMethyl)$_2$ |
| Bis(1-Oxy-2,2,6,6-tetra-methyl)-piperidin-4-yl)-sebacat | O<br>‖<br>HO-P(OEthyl)$_2$ |
| Bis(1-Oxy-2,2,6,6-tetra-methyl)-piperidin-4-yl)-sebacat | O<br>‖<br>HO-P(Oiso-Propyl)$_2$ |
| Bis(1-Oxy-2,2,6,6-tetra-methyl)-piperidin-4-yl)-sebacat | O<br>‖<br>HO-P(OPropyl)$_2$ |
| Bis(1-Oxy-2,2,6,6-tetra-methyl)-piperidin-4-yl)-sebacat | O<br>‖<br>HO-P(On-Butyl)$_2$ |
| Bis(1-Oxy-2,2,6,6-tetra-methyl)-piperidin-4-yl)-sebacat | O<br>‖<br>HO-P(Osec-Butyl)$_2$ |
| Bis(1-Oxy-2,2,6,6-tetra-methyl)-piperidin-4-yl)-sebacat | O<br>‖<br>HO-P(Otert.-Butyl)$_2$ |
| Bis(1-Oxy-2,2,6,6-tetra-methyl)-piperidin-4-yl)-sebacat | O<br>‖<br>HO-P(OPhenyl)$_2$ |
| Bis(1-Oxy-2,2,6,6-tetra-methyl)-piperidin-4-yl)-sebacat | O=P(OH)$_2$(OMethyl) |
| Bis(1-Oxy-2,2,6,6-tetra-methyl)-piperidin-4-yl)-sebacat | O=P(OH)$_2$(OEthyl) |
| Bis(1-Oxy-2,2,6,6-tetra-methyl)-piperidin-4-yl)-sebacat | O=P(OH)$_2$(Oiso-Propyl) |
| Bis(1-Oxy-2,2,6,6-tetra-methyl)-piperidin-4-yl)-sebacat | O=P(OH)$_2$(OPropyl) |
| Bis(1-Oxy-2,2,6,6-tetra-methyl)-piperidin-4-yl)-sebacat | O=P(OH)$_2$(On-Butyl) |

| Inhibitor a) | Inhibitor b) |
|---|---|
| Bis(1-Oxy-2,2,6,6-tetra-methyl)-piperidin-4-yl)-sebacat | $O{=}P(OH)_2$(Osec.-Butyl) |
| Bis(1-Oxy-2,2,6,6-tetra-methyl)-piperidin-4-yl)-sebacat | $O{=}P(OH)_2$(Otert.-Butyl) |
| Bis(1-Oxy-2,2,6,6-tetra-methyl)-piperidin-4-yl)-sebacat | $O{=}P(OH)_2$(OPhenyl) |

Sie eignen sich insbesondere zur Polymerisationsinhibierung von Meth(acrolein), (Meth)acrylsäure und deren Estern.

Beispiele und Vergleichsbeispiele

Allgemeine Versuchsbedingungen

2 ml Acrylsäure wurden jeweils mit verschiedenen Polymerisationsinhibitoren (jeweils X gew.-ppm auf die Acrylsäuremenge bezogen) versetzt und in einer Glasampulle (20 ml Innenvolumen) luftgesättigt gasdicht eingeschlossen.

Dann wurde die Glasampulle in ein 125°C heißes Ölbad so eingetaucht, daß der Flüssigkeitsspiegel der Acrylsäure und der Flüssigkeitsspiegel des Ölbades sich auf gleicher Höhe befanden. Anschließend wurde die Zeit bestimmt, bis die Acrylsäure auspolymerisiert war (der visuell ermittelte Zeitpunkt, zu dem die Acrylsäure verfestigt war).

Die nachfolgende Tabelle zeigt die erhaltenen Ergebnisse.

| Beisp. Bi und Vergleichsbeisp. Vi | Inhibitoren | Zeit [min] |
|---|---|---|
| V 1 | 20 gew.-ppm 4-HO-TEMPO* | 255 |
| V 2 | 20 gew.-ppm Phenothiazin | 265 |
| B 1 | 100 gew.-ppm Irgafos 168,<br>20 gew.-ppm 4-HO-TEMPO | 390 |
| B 2 | 100 gew.-ppm Irgafos P-EPQ,<br>20 gew.-ppm 4-HO-TEMPO | 480 |
| B 3 | 100 gew.-ppm Ultranox 626,<br>20 gew.-ppm 4-HO-TEMPO | 490 |
| B 4 | 100 gew.-ppm $H_3PO_3$,<br>20 gew.-ppm 4-HO-TEMPO | 590 |
| V 3 | 100 gew.-ppm $H_3PO_3$ | 15 |
| V 4 | - | 10 |
| V 5 | 100 gew.-ppm $H_3PO_4$ | 10 |
| B 5 | 20 gew.-ppm 4-HO-TEMPO<br>100 gew.-ppm Phosphorsäure | 520 |
| B 6 | 20 gew.-ppm 4-HO-TEMPO<br>100 gew.-ppm Dimethylphosphit | 525 |
| B 7 | 20 gew.-ppm 4-HO-TEMPO<br>100 gew.-ppm Ammoniumhypophosphit | 520 |
| V 6 | 20 gew.-ppm BTEMPOD**) | 200 |

*) 4-HO-TEMPO = 4-Hydroxy-2,2,6,6-tetramethyl-1-oxyl-piperidin.

**) N,N'-Bis(2,2,6,6-tetramethylpiperidin-1-oxyl-4yl)-N,N'-bis-formyl-1,6-diaminohexan

(fortgesetzt)

| Beisp. Bi und Vergleichsbeisp. Vi | Inhibitoren | Zeit [min] |
|---|---|---|
| B 8 | 20 gew.-ppm BTEMPOD<br>100 gew.-ppm Irgafos 168 | 330 |
| B 9 | 20 gew.-ppm BTEMPOD<br>100 gew.-ppm Ultranox 626 | 495 |
| B 10 | 20 gew.-ppm BTEMPOD<br>100 gew.-ppm $H_3PO_3$ | 660 |
| B 11 | 20 gew.-ppm BTEMPOD<br>100 gew.-ppm Irgafos P-EPQ | 665 |

**Patentansprüche**

1. Verfahren zur Stabilisierung von als Reinsubstanz oder als Bestandteil von Gemischen vorliegenden chemischen Verbindungen, die wenigstens eine ethylenisch ungesättigte Gruppe aufweisen, gegen unerwünschte radikalische Polymerisation, bei dem man der Reinsubstanz oder dem Gemisch Polymerisationsinhibitoren zusetzt, die enthalten:

   a) wenigstens ein Nitroxyl-Radikal (Inhibitoren a))
   und

   b) wenigstens eine das Element Phosphor chemisch gebunden enthaltende chemische Verbindung (Inhibitor b)),

   **dadurch gekennzeichnet, daß** der wenigstens eine Inhibitor b) einerseits keine N-Oxyl-Gruppe und andererseits wenigstens einen Teil des Phosphors mit einer Oxidationszahl OZ $-3 \leq OZ \leq +5$ chemisch gebunden enthält, wobei dieser Phosphor gleichzeitig mit wenigstens einem Wasserstoff, Stickstoff, Sauerstoff und/oder Schwefel kovalent verbunden ist.

2. Verfahren nach Anspruch 1, **dadurch gekennzeichnet, daß** der wenigstens eine Inhibitor b) orthophosphorige Säure, deren Salz und/oder ein Ester derselben ist.

3. Verfahren nach Anspruch 1 oder 2, **dadurch gekennzeichnet, dass** das wenigstens eine Nitroxyl-Radikal eines der allgemeinen Formel I

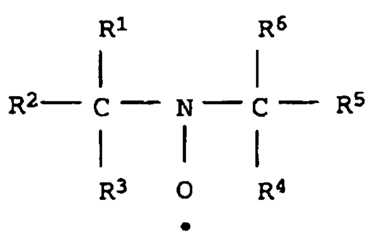

(I),

mit

| | |
|---|---|
| $R^1$,$R^2$,$R^5$ und $R^6$ = | dieselben oder verschiedene gerad- oder verzweigtkettige, gegebenenfalls substituierte Alkylgruppen und |
| $R^3CNCR^4$ = | eine, gegebenenfalls substituierte, zyklische Struktur, |

ist.

4. Verfahren nach Anspruch 1 oder Anspruch 2, **dadurch gekennzeichnet, daß** das wenigstens eine Nitroxyl-Radikal ein Piperidin- und/oder ein Pyrrolidin-N-Oxyl-Radikal ist.

5. Verfahren nach Anspruch 1 oder Anspruch 2, **dadurch gekennzeichnet, daß** das wenigstens eine Nitroxyl-Radikal 4-Hydroxy-2,2,6,6-tetramethyl-1-oxyl-piperidin ist.

6. Verfahren nach einem der Ansprüche 1 bis 5, **dadurch gekennzeichnet, daß** die wenigstens eine ethylenisch ungesättigte Gruppe aufweisende chemische Verbindung wenigstens ein Monomeres aus der Gruppe umfassend Acrylsäure, Methacrylsäure, Acrolein, Methacrolein, Acrylsäureester und Methacrylsäureester ist.

7. Gemische, enthaltend wenigstens ein Nitroxyl-Radikal (Inhibitoren a)) und wenigstens eine das Element Phosphor chemisch gebunden enthaltende chemische Verbindung (Inhibitoren b)), mit der Maßgabe, daß der wenigstens eine Inhibitor b) einerseits keine N-Oxyl-Gruppe und andererseits wenigstens einen Teil des Phosphors mit einer Oxidationszahl OZ $-3 \leq OZ \leq +5$ chemisch gebunden enthält, wobei dieser Phosphor gleichzeitig mit wenigstens einem Wasserstoff, Stickstoff, Sauerstoff und/oder Schwefel kovalent verbunden ist.

8. Gemisch nach Anspruch 7, bei dem der wenigstens eine Inhibitor b) orthophosphorige Säure, deren Salz und/oder ein Ester derselben ist.

9. Gemisch nach Anspruch 7 oder 8, bei dem das wenigstens eine Nitroxyl-Radikal eines der allgemeinen Formel I

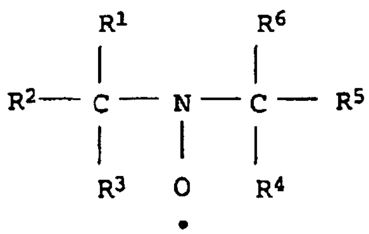

(I),

mit

| | |
|---|---|
| $R^1$,$R^2$,$R^5$ und $R^6$ = | dieselben oder verschiedene gerad- oder verzweigtkettige, gegebenenfalls substituierte Alkylgruppen und |
| $R^3CNCR^4$ = | eine, gegebenenfalls substituierte, zyklische Struktur, |

ist.

10. Gemisch nach Anspruch 7 oder 8, bei dem das wenigstens eine Nitroxyl-Radikal ein Piperidin- und/oder ein Pyrrolidin-N-Oxyl-Radikal ist.

11. Gemisch nach Anspruch 7 oder 8, bei dem das wenigstens eine Nitroxyl-Radikal 4-Hydroxy-2,2,6,6-tetramethyl-1-oxyl-piperidin ist.

12. Verwendung von Gemischen gemäß einem der Ansprüche 7 bis 11 als Polymerisationsinhibitoren für als Reinsubstanz oder als Bestandteil von Gemischen vorliegende Verbindungen, die wenigstens eine ethylenisch ungesättigte Gruppe aufweisen.

13. Gemische, enthaltend wenigstens eine wenigstens eine ethylenisch ungesättigte Gruppe aufweisende Verbindung, wenigstens ein Nitroxyl-Radikal (Inhibitoren a)) und wenigstens eine das Element Phosphor chemisch gebunden enthaltende chemische Verbindung (Inhibitoren b)), mit der Maßgabe, daß der wenigstens eine Inhibitor b) einerseits keine N-Oxyl-Gruppe und andererseits wenigstens einen Teil des Phosphors mit der Oxidationszahl OZ $-3 \leq OZ \leq +5$ chemisch gebunden enthält, wobei dieser Phosphor gleichzeitig mit wenigstens einem Wasserstoff, Stickstoff, Sauerstoff und/oder Schwefel kovalent verbunden ist.

**Claims**

1. A process for stabilising chemical compounds which are present as pure substance or as a component of mixtures and have at least one ethylenically unsaturated group to undesired free radical polymerisation, in which polymerisation inhibitors which contain:

   a) at least one nitroxyl radical (inhibitors a))
   and

   b) at least one chemical compound containing the element phosphorus in chemically bonded form (inhibitor b))

   are added to the pure substance or the mixture, wherein the at least one inhibitor b) contains on the one hand no N-oxyl group and on the other hand at least a part of the phosphorus chemically bonded with an oxidation number ON of -3 ≤ ON s +5, this phosphorus simultaneously being covalently bonded to at least one hydrogen, nitrogen, oxygen and/or sulfur.

2. The process as claimed in claim 1, wherein the at least one inhibitor b) is orthophosphorous acid, a salt thereof and/or an ester thereof.

3. A process as claimed in claim 1 or 2, wherein the at least one N-oxyl radical is of the formula I

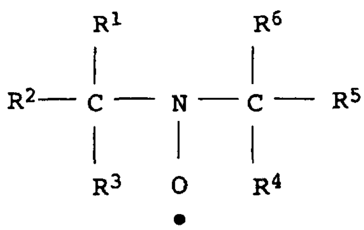

(I),

   where

   | | |
   |---|---|
   | $R^1$,$R^2$,$R^5$ and $R^6$ | are identical or different straight-chain or branched, unsubstituted or substituted alkyl groups and |
   | $R^3CNCR^4$ | is an unsubstituted or substituted cyclic structure. |

4. A process as claimed in claim 1 or claim 2, wherein the at least one nitroxyl radical is a piperidin and/or a pyrrolidin-N-oxyl radical.

5. A process as claimed in claim 1 or claim 2, wherein the at least one nitroxyl radical is 4-hydroxy-2,2,6,6-tetra methyl-1-oxylpiperidine.

6. A process as claimed in any of claims 1 to 5, wherein the chemical compound having at least one ethylenically unsaturated group is at least one monomer from the group consisting of acrylic acid, methacrylic acid, acrolein, methacrolein, acrylates and methacrylates.

7. A mixture containing at least one nitroxyl radical (inhibitors a)) and at least one chemical compound containing the element phosphorus in chemically bonded form (inhibitors b)), with the proviso that the at least one inhibitor b) contains on the one hand no N-oxyl group and on the other hand at least a part of the phosphorus chemically bonded with an oxidation number ON of -3 s ON ≤ +5, this phosphorus simultaneously being covalently bonded to at least one hydrogen, nitrogen, oxygen and/or sulfur.

8. A mixture as claimed in claim 7, in which the at least one inhibitor b) is orthophosphorous acid, a salt thereof and/or an ester thereof.

9. A mixture as claimed in claim 7 or 8, wherein the at least one N-oxyl radical is of the formula I

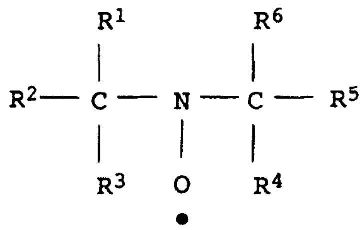 (I),

where

| | |
|---|---|
| $R^1$,$R^2$,$R^5$ and $R^6$ | are identical or different straight-chain or branched, unsubstituted or substituted alkyl groups and |
| $R^3CNCR^4$ | is an unsubstituted or substituted cyclic structure. |

**10.** A mixture as claimed in claim 7 or 8, in which the at least one nitroxyl radical is a piperidin- and/or a pyrrolidin-N-oxyl radical.

**11.** A mixture as claimed in claim 7 or 8, in which the at least one nitroxyl radical is 4-hydroxy-2,2,6,6-tetramethyl-1-oxylpiperidine.

**12.** The use of a mixture as claimed in any of claims 7 to 11 as a polymerisation inhibitor for compounds which are present as pure substance or as a component of mixtures and have at least one ethylenically unsaturated group.

**13.** A mixture containing at least one compound having at least one ethylenically unsaturated group, at least one nitroxyl radical (inhibitors a)) and at least one chemical compound containing the element phosphorus in chemically bonded form (inhibitors b)), with the proviso that the at least one inhibitor b) contains on the one hand no N-oxyl group and on the other hand at least a part of the phosphorus chemically bonded with the oxidation number ON of -3 ≤ ON s +5, this phosphorus simultaneously being covalently bonded to at least one hydrogen, nitrogen, oxygen and/or sulfur.

## Revendications

**1.** Procédé de stabilisation, vis-à-vis d'une polymérisation radicalaire non souhaitée, de composés chimiques se présentant en tant que substance pure ou que constituant de mélange, et contenant au moins un groupe éthyléniquement insaturé, dans lequel on ajoute à la substance pure ou au mélange des inhibiteurs de polymérisation contenant:

a) au moins un radical nitroxyle (inhibiteurs a))
et
b) au moins un composé chimique contenant l'élément phosphore chimiquement lié (inhibiteur b)),

**caractérisé en ce que** le au moins un inhibiteur b) ne contient, d'une part, pas de groupe N-oxyle et, d'autre part, contient au moins une partie du phosphore à un nombre d'oxydation NO -3 ≤ NO ≤ +5 chimiquement lié, ce phosphore étant en même temps lié de manière covalente à au moins un hydrogène, un azote, un oxygène et/ou un soufre.

**2.** Procédé selon la revendication 1, **caractérisé en ce que** le au moins un inhibiteur b) est de l'acide orthophosphoreux, l'un de ses sels et/ou un ester de celui-ci.

**3.** Procédé selon la revendication 1 ou 2, **caractérisé en ce que** le au moins un radical nitroxyle est un radical de la formule générale I

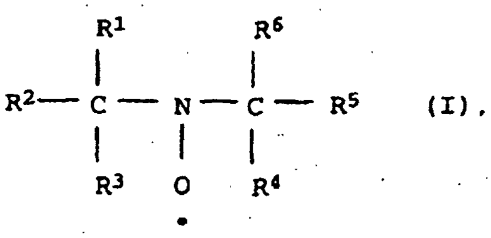

dans laquelle

$R^1$, $R^2$, $R^5$ et $R^6$ sont identiques ou différents et représentent des radicaux alkyle à chaîne linéaire ou ramifiée, éventuellement substitués, et

$R^3$ CNCR$^4$ est une structure cyclique éventuellement substituée.

4. Procédé selon la revendication 1 ou la revendication 2, **caractérisé en ce que** le au moins un radical nitroxyle est un radical pipéridine- et/ou pyrrolidine-N-oxyle.

5. Procédé selon la revendication 1 ou la revendication 2, **caractérisé en ce que** le au moins un radical nitroxyle est un radical 4-hydroxy-2,2,6,6-tétraméthyl-1-oxylpipéridine.

6. Procédé selon l'une quelconque des revendications 1 à 5, **caractérisé en ce que** le composé chimique présentant au moins un groupe éthyléniquement insaturé est au moins un monomère du groupe comprenant l'acide acrylique, l'acide méthacrylique, l'acroléine, la méthacroléine, des esters de l'acide acrylique et des esters de l'acide méthacrylique.

7. Mélanges, contenant au moins un radical nitroxyle (inhibiteurs a)) et au moins un composé chimique contenant l'élément phosphore chimiquement lié (inhibiteurs b)), avec la condition que le au moins un inhibiteur b) ne contienne d'une part pas de groupe N-oxyle et que, d'autre part, il contienne au moins une partie du phosphore à un nombre d'oxydation NO $-3 \leq NO \leq +5$, ce phosphore étant en même temps lié de manière covalente à au moins un hydrogène, un azote, un oxygène et/ou un soufre.

8. Mélange selon la revendication 7, dans lequel le au moins un inhibiteur b) est de l'acide orthophosphoreux, l'un de ses sels et/ou l'un de ses esters.

9. Mélange selon la revendication 7 ou 8, dans lequel le au moins un radical nitroxyle est un radical de la formule générale I

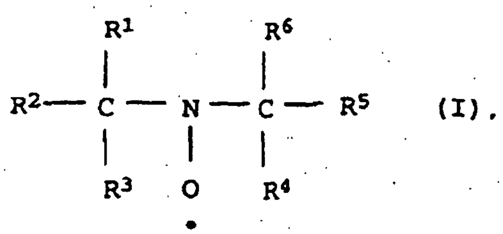

dans laquelle

$R^1$, $R^2$, $R^5$ et $R^6$ sont identiques ou différents et représentent des radicaux alkyle à chaîne linéaire ou ramifiée, éventuellement substitués, et

$R^3$ CNCR$^4$ est une structure cyclique éventuellement substituée.

10. Mélange selon la revendication 7 ou 8, dans lequel le au moins un radical nitroxyle est un radical pipéridine- et/ou pyrrolidine-N-oxyle.

**11.** Mélange selon la revendication 7 ou 8, dans lequel le au moins un radical nitroxyle est un radical 4-hydroxy-2,2,6,6-tétraméthyl-1-oxylpipéridine.

**12.** Utilisation de mélanges selon l'une quelconque des revendications 7 à 11 en tant qu'inhibiteurs de polymérisation pour des composés se présentant en tant que substance pure ou que constituant de mélanges, présentant au moins un groupe éthyléniquement insaturé.

**13.** Mélanges, contenant au moins un composé présentant au moins un groupe éthyléniquement insaturé, au moins un radical nitroxyle (inhibiteurs a)) et au moins un composé chimique contenant l'élément phosphore chimiquement lié (inhibiteurs b)), avec la condition que le au moins un inhibiteur b) ne contienne d'une part pas de groupe N-oxyle et que, d'autre part, il contienne au moins une partie du phosphore à un nombre d'oxydation NO $-3 \leq NO \leq +5$, ce phosphore étant en même temps lié de manière covalente à au moins un hydrogène, un azote, un oxygène et/ou un soufre.